Europäisches Patentamt

(19) European Patent Office

Office européen des brevets

(11) **EP 0 834 570 A1**

(12) **EUROPEAN PATENT APPLICATION**

(43) Date of publication:
08.04.1998 Bulletin 1998/15

(51) Int. Cl.⁶: **C12N 15/52**, C12N 9/12,
C12N 15/70, C12N 1/21,
C12P 21/00

(21) Application number: 96115874.8

(22) Date of filing: 03.10.1996

(84) Designated Contracting States:
CH DE ES FR GB IT LI

(71) Applicant:
BOEHRINGER MANNHEIM GMBH
68298 Mannheim (DE)

(72) Inventors:
• Bonch-Osmolovskaya, Elizaveta Dr.
117192 Moscow (SU)
• Svetlichny, Vitaly Dr.
95448 Bayreuth (DE)
• Ankenbauer, Waltraud, Dr.
82377 Penzberg (DE)

• Schmitz-Agheguian, Gudrun Dr.
82347 Bernried (DE)
• Angerer, Bernhard Dr.
83024 Rosenheim (DE)
• Ebenbichler, Christine
82387 Antdorf (DE)
• Laue, Frank
82396 Paehl (DE)

Remarks:
The applicant has subsequently filed a sequence
listing and declared, that it includes no new matter.

(54) **Thermostable nucleic acid polymerase from Thermococcus gorgonarius**

(57) A purified thermostable enzyme is derived form
the thermophilic archaebacterium *Thermococcus
gorgonarius*. The enzyme can be native or recombinant, retains approximately 90 % of its activity after
incubation for two hours at 95°C in the presence of stabilizing agents and possesses 3′-5′ proofreading exonuclease activity. Thermostable DNA polymerases are
useful in many recombinant DNA techniques, especially
nucleic acid amplification by the polymerase chain reaction (PCR).

EP 0 834 570 A1

Printed by Xerox (UK) Business Services
2.16.1/3.4

## Description

The present invention relates to an extremely thermostable enzyme. More specifically, it relates to a thermostable DNA polymerase obtainable from *Thermococcus gorgonarius*.

DNA polymerases are a family of enzymes which are in particular involved in DNA replication and repair. Extensive research has been conducted on the isolation of DNA polymerases from mesophilic microorganisms such as *E.coli* (see, for example, Bessman et al. (1957) *J. Biol. Chem.* 223:171-177, and Buttin and Kornberg, (1966) *J. Biol. Chem.* 241:5419-5427).

Research has also been conducted on the isolation and purification of DNA polymerases from thermophiles, such as *Thermus aquaticus* (Chien, A., (1976) et al. *J. Bacteriol.* 127:1550-1557) Further, the isolation and purification of a DNA polymerase with a temperature optimum of 80°C from *Thermus aquaticus* YT1 strain has been described (EP 0 258 017 and EP 0 258 017).

Research has indicated that while the Taq DNA polymerase has a 5′-3′ polymerase-dependent exonuclease function, the Taq DNA polymerase does not possess a 3′-5′ proofreading exonuclease function (Lawyer, F.C. et al., (1989) *J. Biol. Chem.*, 264:6427-6437. Bernad A., et al. (1989) *Cell* 59:219). As a result, Taq DNA polymerase is prone to base incorporation errors, making its use in certain applications undesirable. For example, attempting to clone an amplified gene is problematic since any one copy of the gene may contain an error due to a random misincorporation event. Depending on where in the PCR cycle that error occurs (e.g., in an early replication cycle), the entire DNA amplified could contain the erroneously incorporated base, thus, giving rise to a mutated gene product. Furthermore, research has indicated that Taq DNA polymerase has a thermal stability of not more than several minutes at 100°C.

The 3′-5′ exonuclease activity is generally considered to be desirable, because misincorporated or unmatched bases of the synthesized nucleic acid sequence are eliminated by this activity. Therefore, the fidelity of PCR utilizing a polymerase with 3′-5′ exonuclease activity is increased. Such an enzyme is, e.g. the DNA polymerase from Pyrococcus furiosus (Lundberg et al., (1991) *Gene.*, 108; p. 1-6).

Other more recent investigation focusses on the isolation and purification of DNA polymerases from archaebacteria such as *Thermococcus sp.* (EP 0 455 430), in particular a purified DNA polymerase obtainable from *Thermococcus litoralis* is described. Also the recombinant preparation and the gene encoding for this enzyme is known in the art (EP 0 547 920).

In EP 0 455 430 is also described a DNA polymerase from *Pyrococcus sp.* and the gene thereof which also contains introns to be removed for expression of the functional enzyme in *E.coli*.

Accordingly, there is a desire in the art to obtain and produce a purified, highly thermostable DNA polymerase with 3′-5′ proofreading exonuclease activity suitable to improve the PCR process.

The present invention meets this need by providing a DNA polymerase from *Thermococcus gorgonarius* (Tgo) together with the related DNA and amino acid sequence information, recombinant expression vector and a purification protocol for said DNA polymerase. The DNA polymerase according to the present invention exhibits more than a two fold greater replication fidelity than known DNA polymerases, e.g. obtainable from *Pyrococcus furiosus*. A further advantage is that the 3′-5′ exonuclease activity found in *T. gorgonarius* polymerase can also decrease non-specific background amplification in PCR by degrading defrayed ends of primers bound to unspecific sequences thereby destabilizing the binding of the primer because of decreasing the length of the helix. Tgo polymerase is thus unexpectedly superior to known DNA polymerases in amplification protocols requiring high fidelity DNA synthesis. Another advantageous property of the DNA polymerase of *Thermococcus gorgonarius* is the fact, that the gene does not contain intervening sequences which would have to be removed to accomplish expression in *E.coli*.

The thermostable DNA polymerase enzyme obtainable from *T. gorgonarius* catalyzes the template directed polymerization of DNA, has an apparent molecular weight of about 92,000-96,000 daltons and retains 90 % of its activity after incubation for two hours at 95°C in the presence of a stabilizer like a non-ionic detergent as, e. g., 0.01 % Thesit™ (Dodecylpoly(ethylenglycolether)$_n$) or 0.01 % Nonidet P40™ (Ethylphenolpoly(ethylenglycolether)$_n$).

Moreover, DNA encoding the 92,000-96,000 daltons thermostable DNA polymerase obtainable from *Thermococcus gorgonarius* has been isolated and which allows to obtain the thermostable enzyme of the present invention by expression in *E.coli*. The DNA sequence of the DNA polymerase obtainable from *Thermococcus gorgonarius* is shown in SEQ ID No. 6. The recombinant *Thermococcus gorgonarius* DNA polymerase also possesses 3′-5′ exonuclease (proofreading) activity. Furthermore the gene encoding DNA polymerase from *Thermococcus gorgonarius* does not contain intervening sequences.

*Thermococcus gorgonarius* was isolated from E. A. Bonch-Osmolovskaya and V. A. Svetlichny, Institute of Microbiology, Russian Academy of Sciences, Moscow, Russia. *Thermococcus gorgonarius* is a new strain, isolated from a thermal vent in New Zealand. This strain does not show DNA-DNA homology with *T. celer, T. litoralis* or *T. stetteri* (E.A. Bonch-Osmolovskaya, unpublished results).

The preferred thermostable enzyme herein is a DNA polymerase obtainable from *Thermococcus gorgonarius* DSM 8976 (deposited on the Deutsche Sammlung von Mikroorganismen und Zellkulturen GmbH, Mascheroder Weg 1b, D-38124 Braunschweig). This organism is an extrementy thermophilic, sulfur metabolizing, archaebacterium, with a growth range between 55°C and 98°C.

A preferred method for isolation and purification of the enzyme is accomplished - after all growth - using the multistep process as follows:

First, the frozen cells are thawed, suspended in a suitable buffer such as buffer A (40 mM Tris-HCl buffer, pH 7.4; 0.1 mM EDTA, 7 mM 2-mercaptoethanol; 1 mM Pefabloc SC™ (4-(2-Aminoethyl)-benzolsulfonylfluorid)), disrupted by high pressure at 1.200 bar. KCl was added to the extract to a final concentration of 400 mM and the solution cleared by centrifugation. The supernatant is then passed through a Heparin Sepharose Cl 6B column (Pharmacia), which has a strong affinity for nucleic acid binding proteins. The nucleic acids present in the supernatant solution of *Thermococcus gorgonarius* and many of the other proteins pass through the column and are removed by washing the column with two column volumes of buffer A. After washing, the enzyme is eluted with a linear gradient from 0 to 1 M NaCl in buffer A. The peak DNA polymerase activity is dialyzed and applied to a DEAE Sephacel column (Pharmacia). The column is washed with buffer A and the enzyme activity eluted with a linear gradient from 0 to 1 M NaCl in buffer A. The peak DNA polymerase activity is dialyzed and applied to a Cellulose Phosphate column (Whatman). The enzyme is again eluted with a linear gradient such as 0 to 1 M NaCl in buffer A. The enzyme is about 40 % pure at this stage.

The apparent molecular weight of the DNA polymerase obtainable from *Thermococcus gorgonarius* is between about 92,000 to 96,000 daltons when compared with DNA polymerases of known molecular weight, such as *E.coli* DNA polymerase I and *Thermus thermophilus* DNA polymerase. It should be understood, however, that as a protein from an extreme thermophile, *Thermococcus gorgonarius* DNA polymerase may migrate during electrophoresis at an aberrant relative molecular weight due to failure to completely denature or other intrinsic properties. The exact molecular weight of the thermostable enzyme of the present invention may be determined from the coding sequence of the *Thermococcus gorgonarius* DNA polymerase gene. The molecular weight of the DNA polymerase may be determined by any technique , for example, by *in situ* analysis after separation by SDS-polyacrylamide gel electrophoresis (SDS-PAGE) as described in Spanos, A. and Hübscher,U., (1983) *Methods in Enzymology* 91:263-277.

Polymerase activity is either measured by the incorporation of radioactively labeled deoxynucleotides into DNAse-treated, or activated DNA, following subsequent separation of the unincorporated deoxynucleotides from the DNA substrate. Polymerase activity is proportional to the amount of radioactivity in the acid-insoluble fraction comprising the DNA, as described by Lehman, I.R., et al. (1958) *J. Biol. Chem.* 233:163, or by incorporation of digoxigenin-labeled dUTP and determination of incorporated Digoxigenin-dUTP using chemoluminescence according to the method described in Höltke, H.-J.; Sagner, G; Kessler, C.; and Schmitz, G., (1992) *Biotechniques* 12:104 -113.

The DNA polymerase of the present invention has a very high thermal stability at 95°C. It retains approximately 90 percent of its activity after incubation at 95°C for 120 minutes in the presence of stabilizer. The thermal stability is determined by preincubating the enzyme at the temperature of interest in the presence of all assay components (buffer, $MgCl_2$, deoxynucleotides, activated DNA and a stabilizer like 0.01 % Thesit™ and 0.01 % Nonidet P40™) except the single radioactively-labeled deoxynucleotide. At predetermined time intervals, ranging from 1-120 minutes, small aliquots are removed, and assayed for polymerase activity using one of the methods described above.

The thermostable enzyme of this invention may also be produced by recombinant DNA techniques, as the gene encoding this enzyme has been cloned from *Thermococcus gorgonarius* genomic DNA. The complete coding sequence for the *Thermococcus gorgonarius* DNA polymerase can be derived from the plasmid pBTac2Tgo on an approximately 2.3 kB EcoRI/PstI restriction fragment.

The production of a recombinant form of *Thermococcus gorgonarius* DNA polymerase generally includes the following steps: DNA is isolated which codes for the active form of the polymerase. This can be accomplished e.g. by screening of a DNA library derived from the genomic DNA of *T. gorgonarius* using the DNA sequence described in SEQ ID No.: 1 as a probe. Clones containing DNA fragments of *T. gorgonarius* hybridizing to the probe are isolated and the nucleotide sequence of the plasmid inserts determined. Complete isolation of the coding region and the flanking sequences of the DNA polymerase gene can be performed by restriction fragmentation of the *T. gorgonarius* DNA with another restriction enzyme as in the first round of screening and by inverse PCR (Innis et al., (1990) *PCR Protocols*; Academic Press, Inc., p. 219-227). This can be accomplished with synthesized oligonucleotide primers binding at the outer DNA sequences of the gene part but in opposite orientation e.g. with the SEQ ID Nos. 2 and 3. As template *T. gorgonarius* DNA is used which is cleaved by restriction digestion and circularized by contacting with T4 DNA ligase. To isolate the coding region of the whole polymerase gene, another PCR is performed using primers as shown in SEQ ID Nos. 4 and 5 to amplify the complete DNA polymerase gene directly from genomic DNA and introducing ends compatible with the linearized expression vector.

SEQ ID NO. 1:
5'-ATG ATH YTN GAY ACN GAY TAY ATH AC-3'

SEQ ID NO. 2:

5′-GGC CTA CGA GAG GAA CGA ACT GGC-3′

SEQ ID NO. 3:
5′-GGC GTA GAT GTA GGG CTC-3′

SEQ ID NO. 4:
5′-GAG CTG GTC GAA TTC ATG ATC CTG GAC GCT GAC TAC ATC ACC-3′

SEQ ID NO. 5:
5′-AGC CTG CAG TCA TGT CTT AGG TTT TAG CCA CGC-3′

The gene is operably linked to appropriate control sequences for expression in either prokaryotic or eucaryotic host/vector systems. The vector preferably encodes all functions required for transformation and maintenance in a suitable host, and may encode selectable markers and/or control sequences for polymerase expression. Active recombinant thermostable polymerase can be produced by transformed host cultures either continuously or after induction of expression. Active thermostable polymerase can be recovered either from host cells or from the culture media if the protein is secreted through the cell membrane.

It is also preferable that *Thermococcus gorgonarius* thermostable polymerase expression is tightly controlled in *E.coli* during cloning and expression. Vectors useful in practicing the present invention should provide varying degrees of controlled expression of *Thermococcus gorgonarius* polymerase by providing some or all of the following control features: (1) promoters or sites of initiation of transcription, either directly adjacent to the start of the polymerase gene or as fusion proteins, (2) operators which could be used to turn gene expression on or off, (3) ribosome binding sites for improved translation, and (4) transcription or translation termination sites for improved stability. Appropriate vectors used in cloning and expression of *Thermococcus gorgonarius* polymerase include, for example, phage and plasmids. Example of phage include lambda gt11 (Promega), lambda Dash (Stratagene) lambda ZapII (Stratagene). Examples of plasmids include pBR322, pBTac2 (Boehringer Mannheim), pBluescript (Stratagene), pSP73 (Promega), pET3A (Rosenberg, supra, (1987) *Gene* 56:125-135) and pET11C (Studier, F. W. (1990) *Methods in Enzymology*, 185:60-89). According to the present invention the use of a plasmid has shown to be advantageously, particularly pBTac2. The Plasmid pBTac2 carrying the *Anaerocellum thermophilum* DNA polymerase gen is then designated pBTac2Tgo.

Standard protocols exist for transformation, phage infection and cell culture (Maniatis, et al. (1982) *Molecular Cloning: A Laboratory Manual*, Cold Spring Harbour Laboratory Press). Of the numerous *E.coli* strains which can be used for plasmid transformation, the preferred strains include JM110 (ATCC 47013), LE392 pUBS 520 (Maniatis et al. *supra*; Brinkmann et al., (1989) *Gene* 85:109-114;), JM101 (ATCC No. 33876), XL1 (Stratagene), and RR1 (ATCC no. 31343), and BL21 (DE3) plysS (Studier, F. W. et al., (1990) *Methods in Enzymology, supra*). According to the present invention the use of the *E. coli* strain LE392 pUBS 520 has shown to be advantageously. The *E. coli* strain LE392 pUBS 520 transformed with the plasmid pBTac2Tgo is then designated *E. coli* pBtac2Tgo (DSM No. 11178). *E.coli* strain XL1 Blue (Stratagene) is among the strains that can be used for lambda phage, and Y1089 can be used for lambda gt11 lysogeny. The transformed cells are preferably grown at 37°C and expression of the cloned gene is induced with IPTG (Isopropyl-β-D-thiogalactopyranosid).

Isolation of the recombinant DNA polymerase can be performed by standard techniques. Separation and purification of the DNA polymerase from the *E.coli* extract can be performed by standard methods. These methbds include, for example, methods utilizing solubility such as salt precipitation and solvent precipitation, methods utilizing the difference in molecular weight such as dialysis, ultra-filtration, gel-filtration, and SDS-polyacrylamide gel electrophoresis, methbds utilizing a difference in electric charge such as ion-exchange column chromatography, methods utilizing specific interaction such as affinity chromatography, methods utilizing a difference in hydrophobicity such as reversed-phase high performance liquid chromatography and methbds utilizing a difference in isoelectric point such as isoelectric focussing electrophoresis.

One preferred method for isolating and purification of the recombinant enzyme is accomplished using the multi-stage process as follows.

The frozen cells are thawed and suspended in a suitable buffer such as buffer A (40 mM Tris-HCl, pH 7.5; 0.1 mM EDTA; 7 mM 2-mercaptoethanol) in the presence of Pefabloc SC in a final concentration of 1 mM, lysed by the addition of lysozyme (200 µg/ml) under stirring for 30 min. at 4°C. Sodium deoxycholate is added to a final concentration of 0.05 %. After an incubation for another 30 min. KCl is added to a final concentration of 0.75 M. The suspension is incubated at 72°C for 15 min. and centrifuged. The supernatant is adjusted to 25 % saturation with $(NH_4)_2SO_4$ and then applied to a hydrophobic interaction chromatography column such as TSK Butyl Toyopearl 650C (TosoHaas). Most of the nucleic acids and unspecific proteins are in the flow through and wash of the column while the polymerase is eluting at the end of a decreasing gradient from 30 % to 0 % saturation of $(NH_4)_2SO_4$ in buffer A (with additional 10 % glycerol). The polymerase-active fractions are pooled, dialyzed against buffer A containing 10 % glycerol, adjusted to 10 mM

MgCl$_2$ and applied to a high affinity column for nucleotide-binding enzymes such as Fractogel TSK AF-Blue column (Merck). The column is washed with buffer A containing 10 % glycerol and the polymerase protein is eluted with a linear gradient of 0 to 3 M NaCl in buffer A (with additional 10 % glycerol). The polymerase fractions are pooled and dialyzed against the storage buffer B (20 mM Tris-HCl, pH 8.0; 0.1 mM EDTA; 10 mM 2-mercaptoethanol; 50 mM (NH$_4$)$_2$SO$_4$; 50 % glycerol) and stored at -20°C.

The *Thermococcus gorgonarius* DNA polymerase of the present invention may be used for any purpose in which such an enzyme is necessary or desirable. For example, in recombinant DNA technology including, second-strand cDNA synthesis in cDNA cloning and DNA sequencing. See Maniatis, et al., *supra.*

The *Thermococcus gorgonarius* DNA polymerase of the present invention may be modified chemically or genetically - site directed or random - to inactivate the 3′-5′ exonuclease function and used for any purpose in which such a modified enzyme is desirable, e.g., DNA sequencing or DNA labelling.

In addition, the *Thermococcus gorgonarius* DNA polymerase of the present invention may also be used to amplify DNA, e.g., by the procedure disclosed in EP 0 200 362, EP 0 201 184, EP 0 200 362 and EP 0 693 078.

The following examples are given to illustrate embodiments of the present invention as it is presently preferred to practice. It will be understood that the examples are illustrative, and that the invention is not be considered as restricted except as indicated in the appended claims.

Brief description of the drawings

Figure 1:
SDS polyacrylamide gel analysis of partially purified and purified recombinant DNA polymerase from *T. gorgonarius*.

Lane 1: 1 µl of crude extrat.
Lane 2: 5 µl of polymerase fraction obtained after the first chromatography step (TSK Butyl Toyopearl 650C)
Lane 3: 5 µl of fraction obtained after second chromatography step (Fractogel Blue).
Lane 4: 10 µl of fraction obtained after second chromatography step (Fractogel Blue).
Lane 5: 10 units of DNA polymerase from Thermococcus gorgonarius.
Lane 6: Molecular weight markers
Lane 7: 10 units of DNA polymerase from *Pyrococcus woesei*.
Lane 8: Molecular weight markers

Figure 2:
*In situ* activity analysis of native and recombinant *Thermococcus gorgonarius* DNA polymerase in comparison to Klenow fragment, Pol I of *E.coli* and *Thermus thermophilus* DNA polymerase as described in Example I. Native and recombinant *Thermococcus gorgonarius* DNA polymerase have the same electrophoretic mobility.

Figure. 3:
DNA sequence and the deduced amino acid sequence of the gene encoding the DNA polymerase from *Thermococcus gorgonarius.*

Figure. 4:
Determination of heat stability of *T.gorgonarius* polymerase as described in Example V.

Figure. 5:
Analysis of 3′-5′ exonuclease activity as described in Example VI.
Various amounts (units are indicated in the figure) of *T.gorgonarius* DNA polymerase were incubated with DNA fragments in the absence (- dNTPs) and presence (+dNTPs) of desoxynucleotide triphosphates. ctrl1 and 2: Control reactions without DNA polymerase.

The 3′-5′exonuclease activity is dependent on the presence or absence of dNTPs.

Example I

Purification of a thermostable DNA polymerase from *Thermococcus gorgonarius*

*Thermococcus gorgonarius* (DSM 8976) was grown in the medium which was prepared as follows: A mineral solution containing KCl, 325 mg/l; $MgCl_2 \cdot 2 H_2O$, 2.75 mg/l; $MgSO_4 \cdot 7 H_2O$, 3.45 mg/l; $NH_4Cl$, 0.25 mg/l; $CaCl_2 \cdot 2 H_2O$, 0.15 mg/l; $KH_2PO_4$, 0.15 mg/l; NaCl, 18 g/l; $NaHCO_3$, 1 g/l; trace elements, 4 ml/l (Balch et al., (1979) *Microbiol. Rev.* 43:260), vitamins , 4 ml/l (Balch supra,); Rezazurin, 1 mg/l; 0.4 ml/l of a 0.2 % solution of $Fe(NH_2)_2(SO_4)_2 \cdot 7 H_2O$ was boiled and cooled. The following components were added to the final concentrations as indicated: Peptone, 5 g/l; yeast extract, 1 g/l; $Na_2S \cdot 9. H_2O$, 250 mg/l and cystein-HCl, 250 mg/l, the pH was adjusted to 6.2 - 6.4. The incubation temperature was 88°C. The cells were cooled to room temperature, collected by centrifugation and stored at -70°C. 6 g of cells were suspended in 12 ml of buffer A (40 mM Tris-HCl, pH 7.5; 0.1 mM EDTA; 7 mM 2-mercaptoethanol) containing 1 mM Pefabloc SC™ and disrupted by pressure at 1200 bar. KCl was added to a final concentration of 400 mM, dissolved and the solution was centrifugated at 48,200 x g for 30 minutes at 4°C. The supernatant was passed through a 31 ml Heparin Sepharose Cl 6B column (Pharmacia). The column was then washed with 62 ml of buffer B (buffer A containing 10 % glycerol). The column was eluted with a 310 ml linear gradient from 0 to 1.0 M NaCl in buffer B. The DNA polymerase eluted between 30 and 45 mS/cm. The fractions containing DNA polymerase activity were pooled and dialyzed twice against 600 ml buffer B respectively and applied to a 18 ml DEAE Sephacel column (Pharmacia). The column was washed with two column volumes of buffer B, and eluted with a 160 ml linear gradient of 0 to 0.9 M NaCl in buffer B. The polymerase activity eluted between 4 and 14 mS/cm. Fractions were pooled, dialyzed twice against buffer B (200 ml each time) and applied to a 4 ml Cellulose Phosphate P11 column (Whatman). The column was washed with 8 ml of buffer B and the activity eluted with a 40 ml linear gradient of 0 to 1 M NaCl. The active fractions which eluted between 13 and 32 mS/cm were pooled, dialyzed against buffer B containing $(NH_4)_2SO_4$ to 25% saturation and applied to a 4 ml TSK Butyl Toyopearl 650C column (TosoHaas). The column was washed with 8 ml 25% $(NH_4)_2SO_4$-saturated buffer B and eluted with 40 ml of a decreasing gradient of 25 % to 0 % $(NH_4)_2SO_4$-saturated buffer B. The polymerase eluted between 74 and 31 mS/cm, the pool was dialyzed against buffer B and applied to a 4 ml Fractogel TSK AF-Orange column (Merck). The column was washed with 8 ml of buffer B and eluted with a 80 ml linear gradient of 0 to 2.0 M NaCl. The active fractions (between 76 and 104 mS/cm) were pooled and dialyzed against storage buffer C (20 mM Tris-HCl, pH 8.0; 0.1 mM EDTA; 10 mM 2-mercaptoethanol; 50 mM $(NH_4)_2SO_4$; 50 % glycerol) and stored at -20°C. At this step the DNA polymerase was approximately 40 % pure.

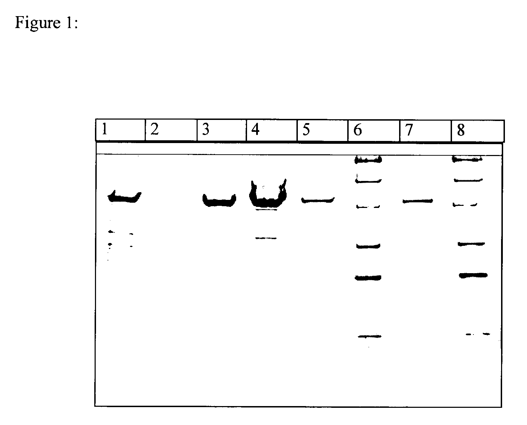

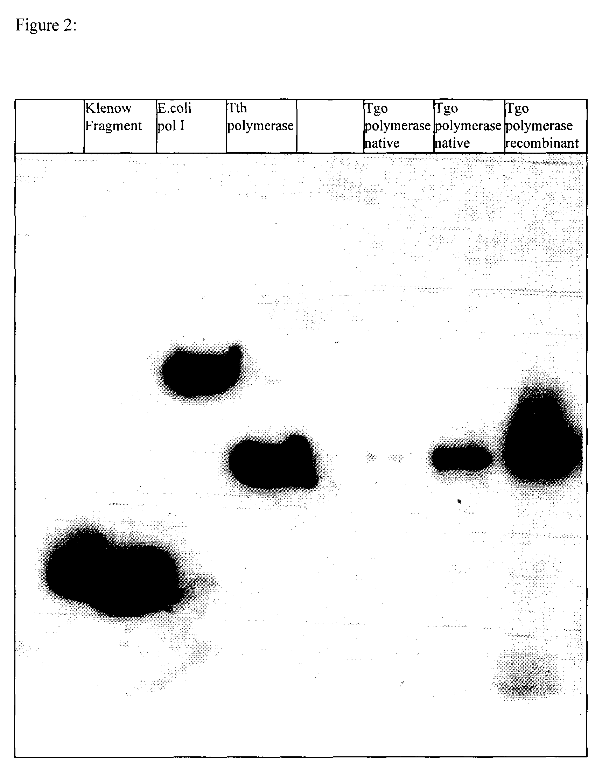

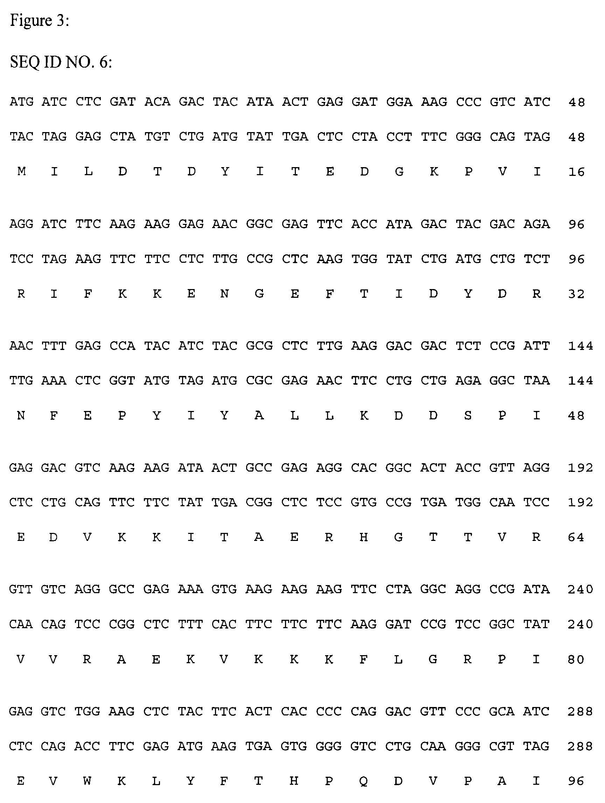

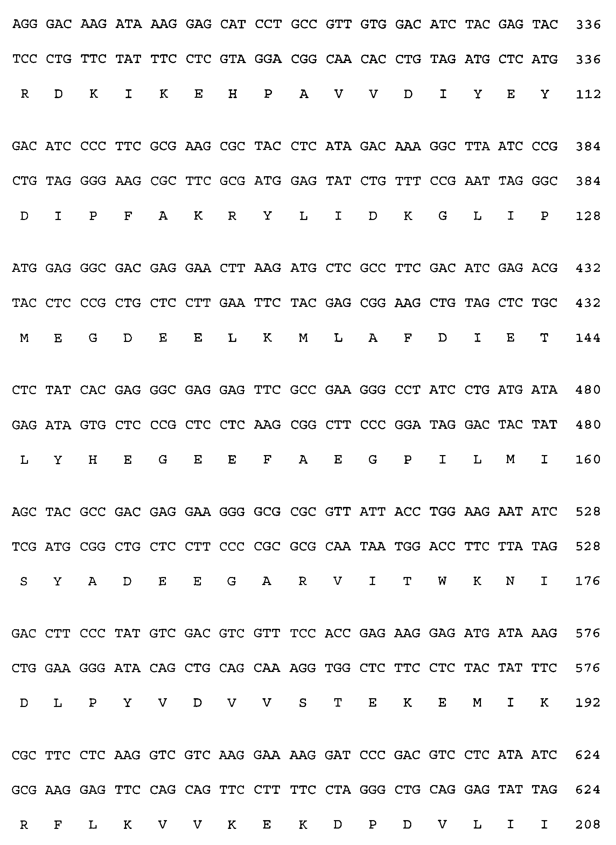

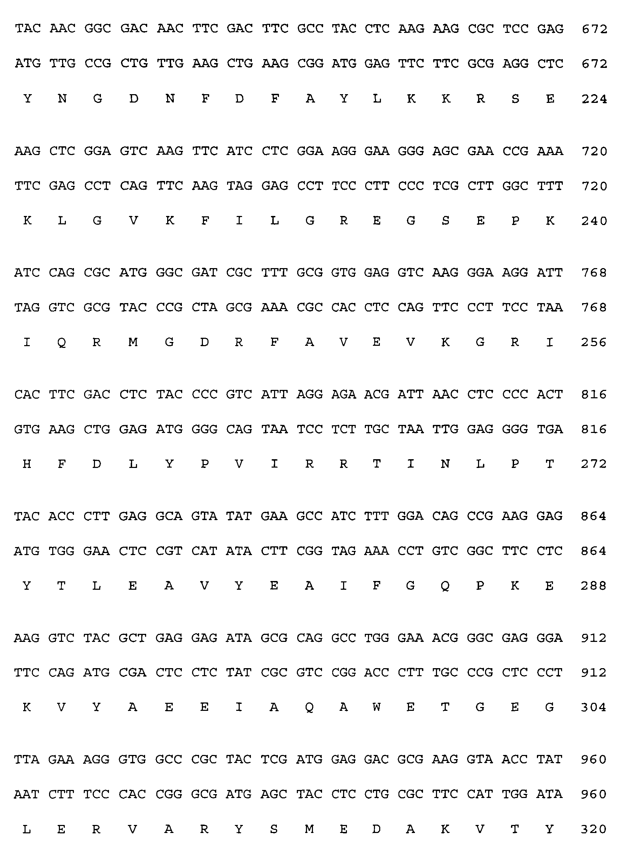

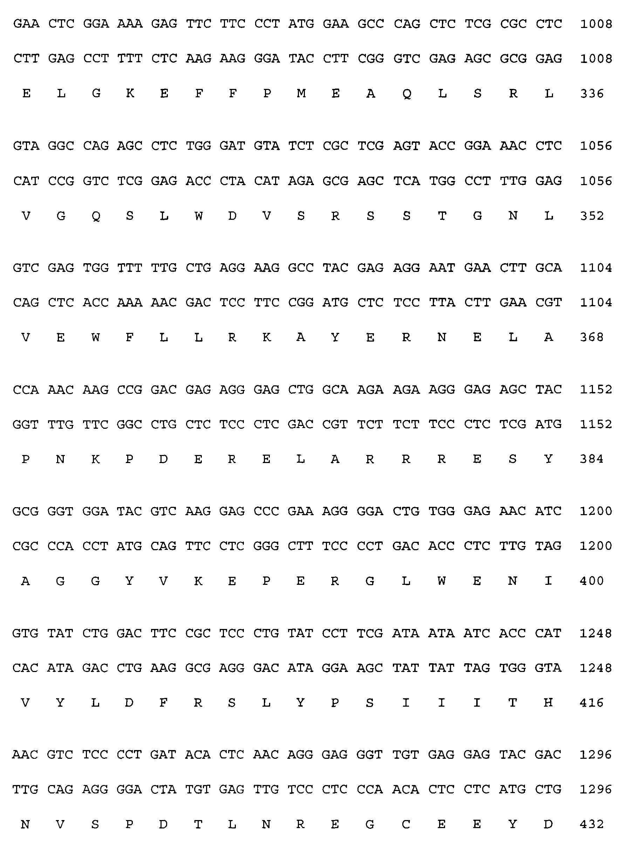

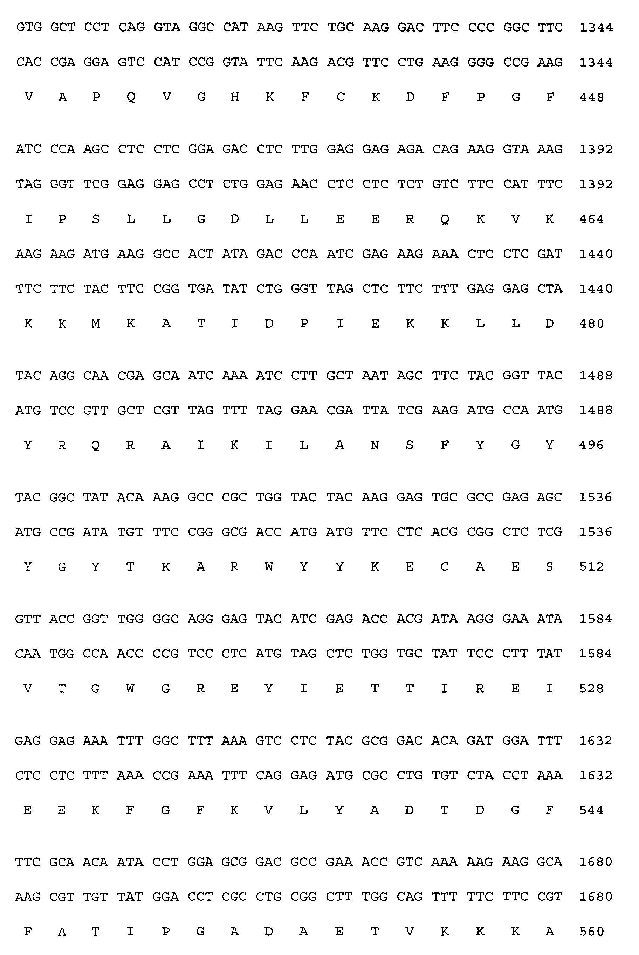

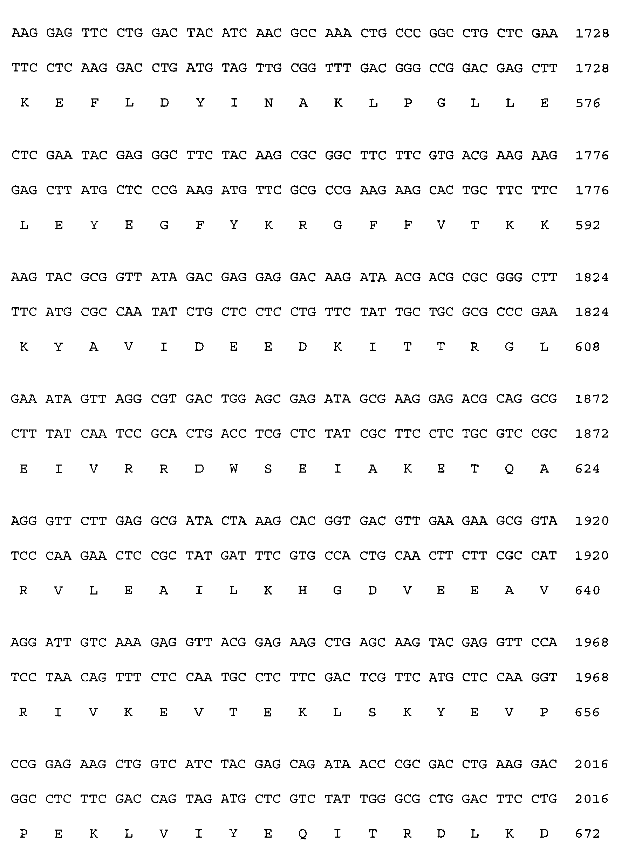

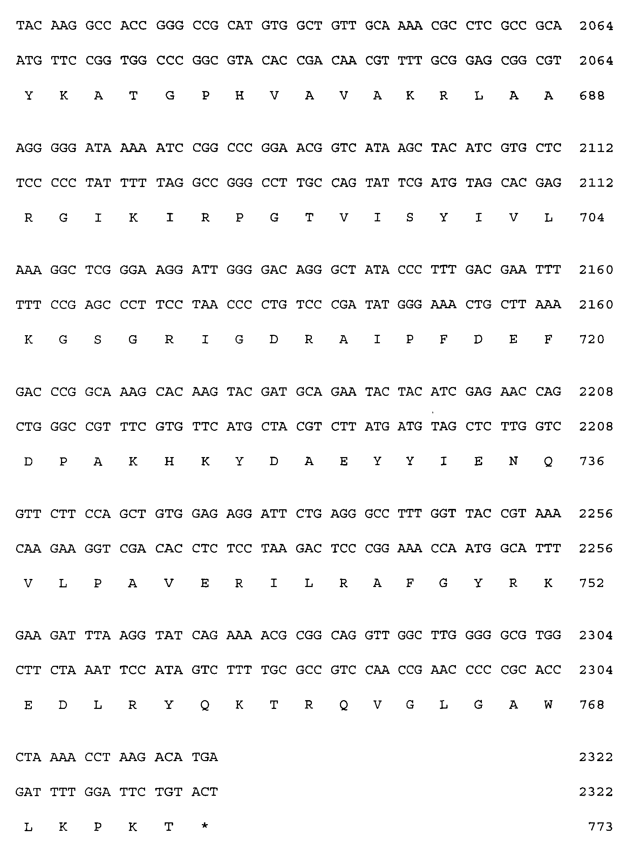

The molecular weight of the isolated DNA polymerase was determined by "activity gel analysis" according to a modified version of the method described by Spanos,A. and Hübscher, U., *supra.* The DNA polymerase sample was separated on a SDS polyacrylamide gel containing activated calf thymus DNA. The polymerase was renaturated in the gel in 50 mM Tris-HCl, pH 8.8; 1 mM EDTA; 3 mM 2-mercaptoethanol; 50 mM KCl; 5 % glycerol. Labeling of the DNA with Dig-dUTP (Boehringer Mannheim) was performed in 10 ml of the following buffer: 50 mM Tris-HCl, pH 8.8; 7 mM $MgCl_2$; 3 mM 2-mercaptoethanol; 100 mM KCl; 12 $\mu$M dGTP; 12 $\mu$M dCTP; 12 $\mu$M dATP; 6 $\mu$M dTTP; 6 $\mu$M Dig-dUTP. The gel was first incubated under shaking at room temperature (30 min.) and then slowly warmed up to 72°C by temperature increments of 5°C. At each temperature interval DNA synthesis is allowed to proceed for 30 min., in order to detect also polymerase activity of mesophile control polymerases. Then the gel was washed and the DNA was blotted on a nylon membrane (Boehringer Mannheim), UV crosslinked. The digoxygenin labeled DNA was detected using the protocol described in the "Boehringer Mannheim's Dig System User's Guide for Filter Hybridization". As molecular weight markers *E.coli* DNA polymerase I, Thermus thermophilus-DNA polymerase and Klenow fragment were analyzed on the same gel. The DNA polymerase isolated from *Thermococcus gorgonarius* has an apparent molecular weight in the range of 92,000 to 96,000 daltons as shown in figure 2.

Example 2

Cloning of the *T. gorgonarius* DNA polymerase

1. DNA from *T. gorgonarius* was isolated and purified by the method described in Lawyer, F. C. et al. (1989) *J. Biol. Chem.* 264:6427-6437.

2. The DNA was restricted with BamHI, separated on an low melting point agarose gel, denatured and blotted onto a nylon membrane. The blot was probed with a Digoxigenin labeled oligonucleotide of the sequence shown in SEQ ID No. 1. A signal could be detected and the region corresponding to the hybridization signal was cut out of the gel. The gel piece was melted and the DNA isolated by ethanol precipitation.

3. The DNA fragments isolated were ligated into a plasmid vector, hybridized with SEQ ID. NO 1. The plasmid DNA from positive clones were isolated and the nucleic acid sequences of the insert determined. The DNA sequences obtained were then compared with sequences of DNA polymerase genes published in Braithwaite, D. K. and Ito J.(1993), *Nucl. Acids Res.* 21:787-802.

4. From the sequence of one of the cloned fragments which showed a high degree of homology to the B type DNA polymerases described in the publication of Braithwaite et al., *supra*, the primers SEQ ID No. 2 and 3 were designed. These primers bind close to the ends of the cloned DNA fragment in opposit orientations to allow amplification of the flanking genomic sequences in circularized template DNA.

5. With these primers "inverse PCR" was performed according of the method of Innis, M. A., *supra*, with the DNA from step 1 which was cleaved with EcoRI and circularized with T4 DNA ligase. With this technique two fragments were generated and the sequences determined. An open reading frame could be identified. The deduced aminoacid sequence showed strong homologies to known DNA polymerases of the pol B type.

6. From the sequence of the DNA fragment identified in step 5 new primers were designed, the sequences are shown in SEQ ID NO. 4 and 5 which were complementary to the start and the end of the reading frame. The primers contained additional non complementary 5' sequences with restriction sites to introduce clonable ends into the PCR product in such an orientation that the product would be under transcriptional and translational control of the promoter.

7. The PCR product was cleaved with EcoRI and PstI, purified and ligated into the vector pBTac2. This clone, expressing the DNA polymerase from *Thermococcus gorgonarius* was designated pBTac2Tgo.

SEQ ID NO. 1:
5'-ATG ATH YTN GAY ACN GAY TAY ATH AC-3'

SEQ ID NO. 2:
5'-GGC CTA CGA GAG GAA CGA ACT GGC-3'

SEQ ID NO. 3:
5'-GGC GTA GAT GTA GGG CTC-3'

SEQ ID NO. 4:
5'-GAG CTG GTC GAA TTC ATG ATC CTG GAC GCT GAC TAC ATC ACC-3'

SEQ ID NO. 5:
5'-AGC CTG CAG TCA TGT CTT AGG TTT TAG CCA CGC-3'

Example 3

Expression of recombinant *T. gorgonarius* DNA

The vector from example 2 was transformed into *E.coli* strain LE 392 pUBS 520, cultivated in a fermentor in a rich medium containing the appropriate antibiotic. Induction was performed at an optical density of 1.25 $A_{540}$ with 0.5 mM IPTG. The DNA polymerase from *T. gorgonarius* may also be cloned and expressed by other methods.

Cells are harvested at an optical density of 5.4 $A_{540}$ by centrifugation and frozen until needed or lyzed by treatment with lysozyme to produce a crude cell extract containing the *T. gorgonarius* DNA polymerase activity.

The crude extract containing the *T. gorgonarius* DNA polymerase activity is purified by the method described in Example 1, or by other purification techniques such as affinity-chromatography, ion-exchange-chromatography or hydrophobic-interaction-chromatography.

Example 4

Purification of recombinate *T. gorgonarius* DNA Polymerase

E.coli (LE392 pUBS520) pBtac2Tgo (DSM No. 11178) was grown in a 10 l fermentor in media containing 20 g/liter tryptone, 10 g/liter yeast extract, 5 g/liter NaCl and 100 mg/liter ampicillin at 37°C and induced with 0.5 mM IPTG at midexponential growth phase and incubated an additional 4 hours. About 45 g of cells were harvested by centrifugation and stored at -70°C.

2 g of cells were thawed and suspended at room temperature in 4 ml of Buffer A (40 mM Tris-HCl, pH 7.5; 0.1 mM EDTA; 7 mM 2-mercaptoethanol; 1 mM Pefabloc SC). 1.2 mg of lysozyme were added and the cells were lyzed under stirring for 30 minutes at 4°C. 4.56 mg sodium deoxycholate were added and the suspension incubated for 10 minutes at room temperature followed by 20 minutes at 0°C. The crude extract was adjusted to 750 mM KCl, heated for 15 minutes at 72°C and centrifuged for removal of denatured protein.

The supernatant was adjusted to 25 % saturation with $(NH_4)_2SO_4$ and applied to a TSK Butyl Toyopearl 650C column (1.5 x 10 cm; 17.7 ml bed volume) equilibrated with buffer B (buffer A containing 10 % glycerol) and 30 % $(NH_4)_2SO_4$-saturation. The column was washed with 70 ml of buffer B and the polymerase was eluted with a 177 ml linear gradient of buffer B containing 30 % to 0 % $(NH_4)_2SO_4$ saturation and 0 to 0.2 % Thesit™ (v/v).

The column fractions were assayed for DNA polymerase activity. DNA polymerase activity was measured by incorporation of digoxigenin labeled dUTP into the newly synthesized DNA and detection and quantification of the incorporated digoxigenin essentially as described below. The reaction is performed in a reaction volume of 50 μl containing 50 mM Tris-HCl, pH 8.5; 15 mM $(NH_4)_2SO_4$; 7 mM $MgCl_2$; 10 mM 2-mercaptoethanol; 100 μM of dATP, dGTP, dCTP, dTTP, respectively; 200 μg/ml BSA; 12 μg of DNAse activated DNA from calf thymus and 0.036 μM digoxigenin-dUTP and 1 or 2 μl of diluted (0.05 U to 0.01 U) DNA polymerase from *T. gorgonarius*. The samples are incubated for 30 min. at 72°C, the reaction is stopped by addition of 2 μl of 0.5 M EDTA, and the tubes placed on ice. After addition of 8 μl of 5 M NaCl and 150 μl of Ethanol (precooled to -20°C) the DNA is precipitated by incubation for 15 min. on ice and pelleted by centrifugation for 10 min. at 13,000 rpm and 4°C. The pellet is washed with 100 μl of 70 % Ethanol (precooled to -20°C) and 0.2 M NaCl, centrifuged again and dried under vacuum. The pellets are dissolved in 50 μl Tris/EDTA (10 mM/0.1 mM; pH 7.5). 5 μl of the sample are spotted into a well of a nylon membrane bottomed white microwell plate (Pall Filtrationstechnik GmbH, Dreieich, FRG, product no: SM045BWP). The DNA is fixed to the membrane by baking for 10 min. at 70°C. The DNA loaded wells are filled with 100 μl of 0.45 μm filtrated 1 % blocking solution (maleic acid, 100 mM; NaCl, 150 mM; casein, 1 % (w/v); pH 7.5). All following incubation steps are done at room temperature. After incubation for 2 min. the solution is sucked through the membrane with a situable vacuum manifold at -0.4 bar. After repeating the washing step once the wells are filled with 100 μl of a 1:10,000-dilution of Anti-digoxigenin-AP Fab fragments (Boehringer Mannheim, FRG, No: 1 093 274) diluted in the blocking solution described above. After incubation for 2 min. and sucking the solution through the membrane, this step is repeated once. The wells are washed twice under vacuum with 200 μl washing-buffer 1 (maleic-acid, 100 mM; NaCl, 150 mM; Tween™ 20, 0.3 % (v/v); pH 7.5) After washing for another two times under vacuum with 200 μl washing-buffer 2 (Tris-HCl, 10 mM; NaCl, 100 mM; $MgCl_2$, 50 mM; pH 9.5) the wells are incubated for 5 min. with 50 μl of CSPD™ (Boehringer Mannheim, No: 1 655 884), diluted 1:100 in washing buffer 2 which serves as a chemiluminescent substrate for the subsequent alkaline phosphatase reaction.

The solution is sucked through the membrane and after 10 min. incubation the RLU/s (Relative Light Unit per second) are detected in a Luminometer e.g. MicroLumat LB 96 P (EG&G Berthold, Wilbad, FRG).

In order to correlate the relative light units to the polymerase units as defined commonly, a standard curve was prepared using a serial dilution of Taq DNA polymerase as a standard enzyme. The Taq polymerase was assayed in the buffer recommended by the supplier. The linear range of the standard curve was used to determine the relative activity of the *T. gorgonarius* DNA polymerase preparations.

The active fractions were pooled, dialyzed twice against 500 ml Buffer B and applied to a Fractogel TSK AF-Blue column (1x10; 7.8 ml bed volume) equilibrated with buffer B. After washing with 15 ml buffer B the column was eluted with a linear gradient of 156 ml from 0 to 3 M NaCl in buffer B supplemented with 0.05 % Thesit. The active fractions were pooled and dialyzed against the storage buffer C (20 mM Tris-HCl, pH 8.2; 10 mM 2-mercaptoethanol; 0.1 mM EDTA; 50 mM $(NH_4)_2SO_4$; 50 % glycerol). After adding of 0.5 % of Nonidet™ P 40 (v/v) and 0.5 % of Thesit™ (v/v) the preparation was stored at -20°C.

Characterisation of the recombinant DNA Polymerase from *Thermococcus gorgonarius*

Recombinant and native *T. gorgonarius* DNA polymerase had the same apparent molecular weight when electrophoresed in 8 - 25 % SDS-PAGE gradient gels. Recombinant *T. gorgonarius* polymerase maintains the heat stability of the native enzyme. Recombinant *T. gorgonarius* polymerase has the same 3'-5'exonuclease activity as native *T. gorgonarius* polymerase, which is also sensitive to inhibition by an excess of dNTPs.

Example 5

Thermostability of *T. gorgonarius* DNA Polymerase

The thermostability of the DNA polymerase from *T. gorgonarius* purified as described in Example I was determinated as follows: 5 units purified *T. gorgonarius* polymerase were incubated at 95°C in 100 μl of the following buffer: 50 mM Tris-HCl, pH 8.8 (at 25°C); 15 mM $(NH_4)_2SO_4$; 7 mM $MgCl_2$; 10 mM 2-mercaptoethanol; 200 μM each of dATP, dGTP, dCTP and dTTP; 0.1 % Nonidet P40, 0.1 % Thesit; 25 μg DNAse treated calf thymus DNA. 15 μl samples were taken at 0, 5, 10, 15, 30, 45, 60 and 120 minutes. The remaining polymerase activity was measured as described in example IV by determining incorporation of labeled $^3$H-TTP into DNA in a 50 μl volume of the incubation mixture described above containing in addition 150 nCi of $^3$H-TTP. After incubation at 72°C for 30 minutes the reactions were stopped by addition of 300 μl 10 % TCA, and after 10 minutes at 0°C the mixtures were applied onto 3MM filters (Whatman). The filters were washed three times with approximately 10 ml 5 % TCA each time, dried for 10 minutes at 75°C and the DNA bound radioactivity of each filter was measured in 5 ml scintillation liquid in a scintillation vial in LKB rack beta 1217/1218 (Pharmacia).

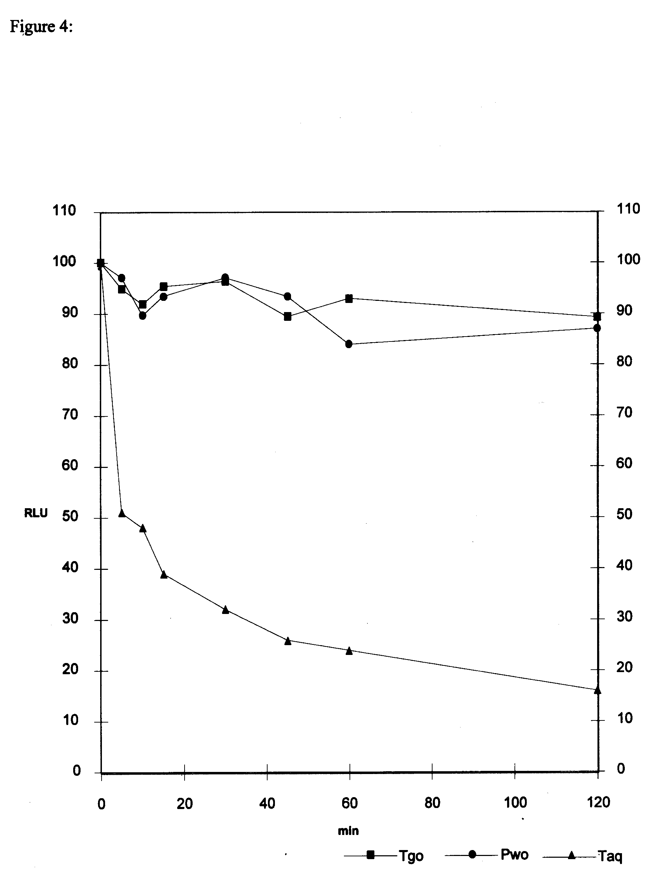

As shown in figure No. 4 the *T. gorgonarius* DNA polymerase retained almost 90 % of its initial activity after incubation for 120 minutes at 95°C, Pwo polymerase has a similar stability, while Taq DNA polymerase has a remaining activity of approximately 16 % only.

Example 6

Determination of 3′-5′ proofreading activity

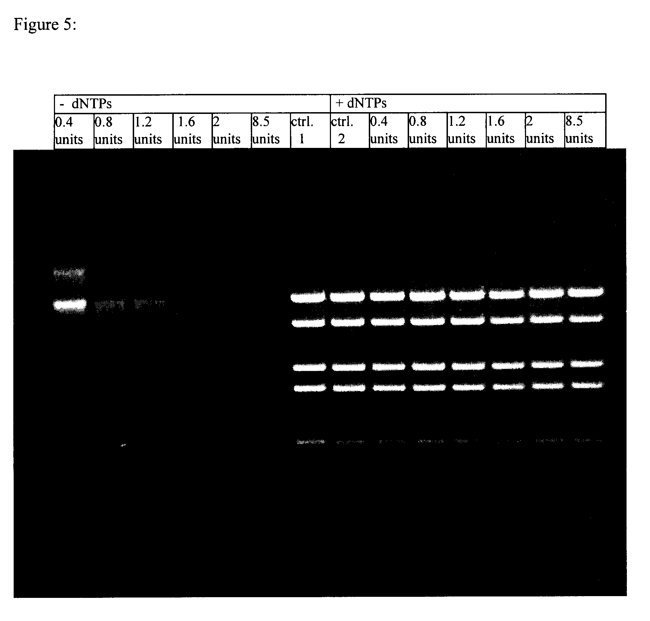

A series of units of *T. gorgonarius* DNA polymerase (see figure 5) were incubated for 4 hours at 72°C with 1 μg DNA molecular weight marker VI (Boehringer Mannheim) in the presence and absence of dNTP's, 1 mM each, in 50 μl of the following incubation buffer: 50 mM Tris-HCl, pH 7.8; 10 mM $MgCl_2$; 7 mM 2-mercaptoethanol with Paraffin overlay. After addition of 10 μl stop solution the DNA fragments were separated on a 1 % agarose gel. In the absence of dNTP's a smear of DNA fragments or no DNA could be detected while in presence of dNTP's the DNA fragments remained undegraded.

Example 7

Fidelity of *T. gorgonarius* DNA polymerase in the PCR process

The fidelity of *T. gorgonarius* DNA polymerase in the PCR process was determined in an assay based on the amplification, circularisation and transformation of the pUC19 derivate pUCIQ17, containing a functional *lac* I[q] allele (Frey, B. and Suppmann B. (1995) *Biochemica* 2:34-35). PCR-derived mutations in *lac* I are resulting in a derepression of the expression of *lac* Zα and subsequent formation of a functional β-galactosidase enzyme which can be easily detected on X-Gal indicator plates . The error rates determined with this *lac* I-based PCR fidelity assay were in the range of 3.4 to 2.2 • $10^{-6}$.

The plasmid pUCIQ17 was linearized by digestion with DraII to serve as a substrate for PCR amplification with DNA polymerase of *T. gorgonarius*. Both of the primers used have ClaI sites at their 5 prime ends:

Primer 1: 5′-AGCTTATCGATGGCACTTTTCGGGGAAATGTGCG-3′
Primer 2: 5′-AGCTTATCGATAAGCGGATGCCGGGAGCAGACAAGC-3′

The length of the resulting PCR product is 3493 pb.

The PCR was performed in a final volume of 50 μl in the presence of 1.5 mM $MgCl_2$, 50 mM Tris⁻HCl, pH 8.5 (25°C), 12.5 mM $(NH_4)_2SO_4$, 35 mM KCl, 200 μM dNTPs and 2.5 units of *T. gorgonarius* DNA polymerase. Conditions of the amplification reaction using *T.gorgonarius* DNA polymerase are

The cycle conditions were as follows:

1 x    denaturation of template for 2 min. at 95°C

$$
8\ \text{x}\ \left[\begin{array}{l}
\text{denaturation at 95°C for 10 sec.} \\
\text{annealing at 57°C for 30 sec.} \\
\text{elongation at 72°C for 4 min.}
\end{array}\right.
$$

$$
16\ \text{x}\ \left[\begin{array}{l}
\text{denaturation at 95°C for 10 sec.} \\
\text{annealing at 57°C for 30 sec.} \\
\text{elongation at 72°C for 4 min.} \\
\text{+ cycle elongation of 20 sec. for each cycle}
\end{array}\right.
$$

After PCR, the PCR products were PEG-precipitated (Barnes, W. M. (1992) *Gene* 112:229) the DNA restricted with ClaI and purified by agarose gel electrophoresis. The isolated DNA was ligated using the Rapid DNA Ligation Kit (Boehringer Mannheim GmbH) and the ligation products transformed in *E.coli* DH5α, plated on TN Amp X-Gal plates. The α-complementing *E.coli* strain DH5α transformed with the resulting plasmid pUCIQ17 (3632 bp), shows white (lacl⁺) colonies on TN plates (1.5 % Bacto Tryptone, 1 % NaCl, 1.5 % Agar) containing ampicillin (100 μg/ml) and X-Gal (0.004 % w/v). Mutations result in blue colonies.

After incubation overnight at 37°C, blue and white colonies were counted. The error rate (f) per bp was calculated with a rearranged equation as published by Keohavong and Thilly (Keohavong, P. and Thilly, W. (1989) *PNAS* USA 86:9253):

$$f = \text{-lnF} \ / \ d \ \text{x} \ b \ \text{bp}$$

where **F** is the fraction of white colonies:

F = white (lacl+) colonies / total colony number;

**d** is the number of DNA duplications:

$$2^d = \text{output DNA / input DNA;}$$

and **b** is the effective target size of the (1080bp) *lac* I gene, which is 349 bp according to Provost et al. (Provost et al. (1993) *Mut. Res.* 288:133).

Example 8

Fidelity assay

Determination of the misincorporation rates of DNA polymerases from *Pyrococcus furiosus* and *Thermococcus gorgonarius* under PCR conditions.

Error rates of many DNA polymerases are published. For example for the DNA polymerase of *Pyrococcus furiosus* various error rates were measured (Lit. 1-5). They may vary with the conditions used e.g. nucleotide triphosphate concentrations, enzyme preparation, buffer conditions and of course with the method used, the determination of the number of duplications and the way to calculate the misincorporation rate.

Therefore, the DNA polymerases *Pfu* (Stratagene) and *Tgo* (Boehringer Mannheim GmbH) were analyzed in parallel in the same system (Protocol: Frey, B. and Suppman, B. Boehringer Mannheim Biochemica Information, Nr. 96-1995, 21-23).

Table 1:

Fidelity of *Pfu* and *Tgo* DNA polymerases in PCR fidelity assay

| DNA Polymerase | Plaques scored | | Mutation | Error rate (a) | Error rate (b) |
|---|---|---|---|---|---|
| | Total | Mutant | frequency | | |
| *Pfu* | | | | | |
| 1. sample | 3082 | 76 | 2.47 | $1,56 \times 10^{-5}$ | $8,2 \times 10^{-6}$ |
| 2. sample | 2693 | 68 | 2.52 | $1,6 \times 10^{-5}$ | $8,4 \times 10^{-6}$ |
| *Tgo* | | | | | |
| 1. sample | 1904 | 12 | 0.63 | $3,5 \times 10^{-6}$ | $1,8 \times 10^{-6}$ |
| 2. sample | 2003 | 20 | 1 | $5,6 \times 10^{-6}$ | $2,9 \times 10^{-6}$ |

(a) Error rate calculated according to the equation used by Stratagene (Lundberg K.S. et al. (1991) Gene <u>108</u>, 1-6).

$$ER = mf / bp \times d$$

ER = error rate

mf = mutation frequency in % minus background frequency of 0.0017 %

bp is the number of detectable sites in the *lac I* gene sequence (182)

d is the number of duplications. In this particular experiment the number of duplications was determined/estimate for *Pfu* to be 8,64 and for *Tgo* to be 9,64

(b) Error rate calculated per bp with a rearranged equation published by Keohavong P. and Thilly W. (1989) *PNAS* USA **86**, 9253.

$$ER = -INF / d \times b \text{ bp\#}$$

F = fraction of white colonies (white colonies / total number of colonies)

d = the number of duplications. $2^d$ = output DNA / input DNA

b is the effective target size of the (1080 bp) *lac I* gene, which is 349 bp according to Provost, G.S., Kretz, P.L., Hammer, R.T., Matthews, C.D., Rogers, B.J., Lundberg K., S., Dycaico, M.J. and Short, J.M. (1993) *Mut. Res.* **288**, 133 ff.

Result:

These data show that the mutation frequency of *Tgo* DNA polymerase is lower than that of *Pfu*, and the fidelity (calculated in errors per base pair) is higher no matter which way of calculation was used.

References describing error rates for *Pfu*:

1. Lundberg, K.S., Shoemaker, D.D., Adams M.W.W:, Short, J.M., Sorge, J.A. and 2 Mathur, E.J. (1991) *Gene* **108**, 1-6. (1.6 x 10-6 errors/base)

2. Flaman, J.-M., Frebourg, T., Moreau, V., Charbonnier, F., Martin, C., Ishioka, C., Friend, S.H. and Iggo, R. (1994) *NAR* **22**, 3259-3260. (2 x 1β-6 errors/base) Für Tli (Vent) Polymerase: (Variations in error rate depending on assay)

3. Cariello, N.F., Swenberg, J.A. and Skopek, T.R. (1991) *NAR* **19**, 4193-4198. (2.4 x 10-5 errors/base)

4. Ling, L.L., Keohavong, P., Dias, C. and Thilly, W.G. (1991) *PCR Methods Appl.* **1**, 63-69. (4.5 x 10-5 errors/base)

5. Matilla, P., Korpela, J., Tenkanen, T. and Pitkanen, K. (1991) *NAR* **19**, 4967-4973. (5.7 x 10-5 errors/base)

## Claims

1. A purified thermostable DNA polymerase obtainable from *Thermococcus gorgonarius*.

2. A purified thermostable DNA polymerase according to claim 1 which catalyses the template directed polymerisation of DNA and retains about 90 % of its activity after incubation for two hours at about 95°C in the presence of a stabilizer.

3. Enzyme according to claims 1 or 2, wherein said polymerase is characterized by a at least a two-fold greater replication fidelity than DNA polymerase obtainable from *Pyrococcus furiosus*.

4. The polymerase as claimed in any one of claims 1 - 3, wherein said polymerase has an apparent molecular weight between about 92 000 to 96 000 daltons.

5. The polymerase as claimed in any one of claims 1 - 4, wherein said polymerase is obtainable from E.coli.

6. A stabilized composition consisting of a polymerase as claimed in any one of claims 1 - 5 and a stabilizer.

7. The composition according to claims 1 - 6, wherein said stabilizer is a non-ionic detergent.

8. The polymerase as claimed in any one of claims 1 - 7, wherein said polymerase is characterized by having 3'-5'-exonuclease (proofreading) activity.

9. The composition according to claim 1 - 8, wherein Thesit and/or Nonident P 40 serve as stabilizer.

10. An isolated DNA sequence coding for the polymerase according to any one of claims 1 - 9 obtainable from *Thermococcus gorgonarius.*

11. An isolated DNA sequence coding for the polymerase as claimed in claim 10 contained within the plasmid pBTac2Tgo.

12. An isolated DNA sequence of claim 11 contained within an approximately 2.3 kB EcoRI/PstI restriction fragment of

plasmid pBTac2Tgo.

13. An isolated DNA sequence represented by the formula shown in SEQ ID NO 6.

14. A vector containing the isolated DNA sequence as claimed in any one of claims 10 - 13.

15. The vector of claim 14, wherein such vector is plasmid pBTac2Tgo.

16. The vector according to claims 14 and 15 providing some or all of the following features:

   (1) promotors or sites of initiation of transcription
   (2) operators which could be used to turn gene expression on or off
   (3) ribosome binding sites for improved translation
   (4) transcription or translation termination sites.

17. A microbial host transformed with the vector of claims 14 - 16.

18. A microbial host according to claim 17 wherein said host is from *E. coli* LE 392 pUBS 520 and designated *E.coli* pBtac2Tgo.

19. A process for the preparation of DNA polymerase according to any one of claims 1 - 9 comprising the steps:

   (a) cultering the natural strain *Thermococcus gorgonarius*
   (b) suspending the cells of the natural cells in buffer
   (c) disrupting the cells
   (d) purifying the DNA polymerase by several chromatographic steps.

20. A process for the preparation of DNA polymerase according to any one of claims 1 - 9 comprising growing a microbial host strain according to claims 17 or 18 and purifying the DNA polymerase therefrom.

21. A process for amplifying DNA, characterized in that a thermostable DNA polymerase according to any one of claims 1 - 9 is used.

22. A process for DNA sequencing or DNA labelling, characterized in that a thermostable DNA polymerase wherein the 3'-5' exonuclease activity of said DNA polymerase is inactivated.

23. A process for second cDNA cloning and DNA sequencing, characterized in that a thermostable DNA polymerase according to any one of claims 1-9 is used.

24. A process for DNA sequencing, characterized in that a thermostable DNA polymerase according to any one of claims 1-9 is used.

28

Figure 1:

Figure 2:

| | Klenow Fragment | E.coli pol I | Tth polymerase | | Tgo polymerase native | Tgo polymerase native | Tgo polymerase recombinant |
|---|---|---|---|---|---|---|---|

Figure 3:

SEQ ID NO. 6:

```
ATG ATC CTC GAT ACA GAC TAC ATA ACT GAG GAT GGA AAG CCC GTC ATC   48

TAC TAG GAG CTA TGT CTG ATG TAT TGA CTC CTA CCT TTC GGG CAG TAG   48

 M   I   L   D   T   D   Y   I   T   E   D   G   K   P   V   I    16


AGG ATC TTC AAG AAG GAG AAC GGC GAG TTC ACC ATA GAC TAC GAC AGA   96

TCC TAG AAG TTC TTC CTC TTG CCG CTC AAG TGG TAT CTG ATG CTG TCT   96

 R   I   F   K   K   E   N   G   E   F   T   I   D   Y   D   R    32


AAC TTT GAG CCA TAC ATC TAC GCG CTC TTG AAG GAC GAC TCT CCG ATT   144

TTG AAA CTC GGT ATG TAG ATG CGC GAG AAC TTC CTG CTG AGA GGC TAA   144

 N   F   E   P   Y   I   Y   A   L   L   K   D   D   S   P   I    48


GAG GAC GTC AAG AAG ATA ACT GCC GAG AGG CAC GGC ACT ACC GTT AGG   192

CTC CTG CAG TTC TTC TAT TGA CGG CTC TCC GTG CCG TGA TGG CAA TCC   192

 E   D   V   K   K   I   T   A   E   R   H   G   T   T   V   R    64


GTT GTC AGG GCC GAG AAA GTG AAG AAG AAG TTC CTA GGC AGG CCG ATA   240

CAA CAG TCC CGG CTC TTT CAC TTC TTC TTC AAG GAT CCG TCC GGC TAT   240

 V   V   R   A   E   K   V   K   K   K   F   L   G   R   P   I    80


GAG GTC TGG AAG CTC TAC TTC ACT CAC CCC CAG GAC GTT CCC GCA ATC   288

CTC CAG ACC TTC GAG ATG AAG TGA GTG GGG GTC CTG CAA GGG CGT TAG   288

 E   V   W   K   L   Y   F   T   H   P   Q   D   V   P   A   I    96
```

```
AGG GAC AAG ATA AAG GAG CAT CCT GCC GTT GTG GAC ATC TAC GAG TAC   336

TCC CTG TTC TAT TTC CTC GTA GGA CGG CAA CAC CTG TAG ATG CTC ATG   336

 R   D   K   I   K   E   H   P   A   V   V   D   I   Y   E   Y    112


GAC ATC CCC TTC GCG AAG CGC TAC CTC ATA GAC AAA GGC TTA ATC CCG   384

CTG TAG GGG AAG CGC TTC GCG ATG GAG TAT CTG TTT CCG AAT TAG GGC   384

 D   I   P   F   A   K   R   Y   L   I   D   K   G   L   I   P    128


ATG GAG GGC GAC GAG GAA CTT AAG ATG CTC GCC TTC GAC ATC GAG ACG   432

TAC CTC CCG CTG CTC CTT GAA TTC TAC GAG CGG AAG CTG TAG CTC TGC   432

 M   E   G   D   E   E   L   K   M   L   A   F   D   I   E   T    144


CTC TAT CAC GAG GGC GAG GAG TTC GCC GAA GGG CCT ATC CTG ATG ATA   480

GAG ATA GTG CTC CCG CTC CTC AAG CGG CTT CCC GGA TAG GAC TAC TAT   480

 L   Y   H   E   G   E   E   F   A   E   G   P   I   L   M   I    160


AGC TAC GCC GAC GAG GAA GGG GCG CGC GTT ATT ACC TGG AAG AAT ATC   528

TCG ATG CGG CTG CTC CTT CCC CGC GCG CAA TAA TGG ACC TTC TTA TAG   528

 S   Y   A   D   E   E   G   A   R   V   I   T   W   K   N   I    176


GAC CTT CCC TAT GTC GAC GTC GTT TCC ACC GAG AAG GAG ATG ATA AAG   576

CTG GAA GGG ATA CAG CTG CAG CAA AGG TGG CTC TTC CTC TAC TAT TTC   576

 D   L   P   Y   V   D   V   V   S   T   E   K   E   M   I   K    192


CGC TTC CTC AAG GTC GTC AAG GAA AAG GAT CCC GAC GTC CTC ATA ATC   624

GCG AAG GAG TTC CAG CAG TTC CTT TTC CTA GGG CTG CAG GAG TAT TAG   624

 R   F   L   K   V   V   K   E   K   D   P   D   V   L   I   I    208
```

```
TAC AAC GGC GAC AAC TTC GAC TTC GCC TAC CTC AAG AAG CGC TCC GAG   672

ATG TTG CCG CTG TTG AAG CTG AAG CGG ATG GAG TTC TTC GCG AGG CTC   672

 Y   N   G   D   N   F   D   F   A   Y   L   K   K   R   S   E    224


AAG CTC GGA GTC AAG TTC ATC CTC GGA AGG GAA GGG AGC GAA CCG AAA   720

TTC GAG CCT CAG TTC AAG TAG GAG CCT TCC CTT CCC TCG CTT GGC TTT   720

 K   L   G   V   K   F   I   L   G   R   E   G   S   E   P   K    240


ATC CAG CGC ATG GGC GAT CGC TTT GCG GTG GAG GTC AAG GGA AGG ATT   768

TAG GTC GCG TAC CCG CTA GCG AAA CGC CAC CTC CAG TTC CCT TCC TAA   768

 I   Q   R   M   G   D   R   F   A   V   E   V   K   G   R   I    256


CAC TTC GAC CTC TAC CCC GTC ATT AGG AGA ACG ATT AAC CTC CCC ACT   816

GTG AAG CTG GAG ATG GGG CAG TAA TCC TCT TGC TAA TTG GAG GGG TGA   816

 H   F   D   L   Y   P   V   I   R   R   T   I   N   L   P   T    272


TAC ACC CTT GAG GCA GTA TAT GAA GCC ATC TTT GGA CAG CCG AAG GAG   864

ATG TGG GAA CTC CGT CAT ATA CTT CGG TAG AAA CCT GTC GGC TTC CTC   864

 Y   T   L   E   A   V   Y   E   A   I   F   G   Q   P   K   E    288


AAG GTC TAC GCT GAG GAG ATA GCG CAG GCC TGG GAA ACG GGC GAG GGA   912

TTC CAG ATG CGA CTC CTC TAT CGC GTC CGG ACC CTT TGC CCG CTC CCT   912

 K   V   Y   A   E   E   I   A   Q   A   W   E   T   G   E   G    304


TTA GAA AGG GTG GCC CGC TAC TCG ATG GAG GAC GCG AAG GTA ACC TAT   960

AAT CTT TCC CAC CGG GCG ATG AGC TAC CTC CTG CGC TTC CAT TGG ATA   960

 L   E   R   V   A   R   Y   S   M   E   D   A   K   V   T   Y    320
```

```
GAA CTC GGA AAA GAG TTC TTC CCT ATG GAA GCC CAG CTC TCG CGC CTC   1008

CTT GAG CCT TTT CTC AAG AAG GGA TAC CTT CGG GTC GAG AGC GCG GAG   1008

 E   L   G   K   E   F   F   P   M   E   A   Q   L   S   R   L    336


GTA GGC CAG AGC CTC TGG GAT GTA TCT CGC TCG AGT ACC GGA AAC CTC   1056

CAT CCG GTC TCG GAG ACC CTA CAT AGA GCG AGC TCA TGG CCT TTG GAG   1056

 V   G   Q   S   L   W   D   V   S   R   S   S   T   G   N   L    352


GTC GAG TGG TTT TTG CTG AGG AAG GCC TAC GAG AGG AAT GAA CTT GCA   1104

CAG CTC ACC AAA AAC GAC TCC TTC CGG ATG CTC TCC TTA CTT GAA CGT   1104

 V   E   W   F   L   L   R   K   A   Y   E   R   N   E   L   A    368


CCA AAC AAG CCG GAC GAG AGG GAG CTG GCA AGA AGA AGG GAG AGC TAC   1152

GGT TTG TTC GGC CTG CTC TCC CTC GAC CGT TCT TCT TCC CTC TCG ATG   1152

 P   N   K   P   D   E   R   E   L   A   R   R   R   E   S   Y    384


GCG GGT GGA TAC GTC AAG GAG CCC GAA AGG GGA CTG TGG GAG AAC ATC   1200

CGC CCA CCT ATG CAG TTC CTC GGG CTT TCC CCT GAC ACC CTC TTG TAG   1200

 A   G   G   Y   V   K   E   P   E   R   G   L   W   E   N   I    400


GTG TAT CTG GAC TTC CGC TCC CTG TAT CCT TCG ATA ATA ATC ACC CAT   1248

CAC ATA GAC CTG AAG GCG AGG GAC ATA GGA AGC TAT TAT TAG TGG GTA   1248

 V   Y   L   D   F   R   S   L   Y   P   S   I   I   I   T   H    416


AAC GTC TCC CCT GAT ACA CTC AAC AGG GAG GGT TGT GAG GAG TAC GAC   1296

TTG CAG AGG GGA CTA TGT GAG TTG TCC CTC CCA ACA CTC CTC ATG CTG   1296

 N   V   S   P   D   T   L   N   R   E   G   C   E   E   Y   D    432
```

```
GTG GCT CCT CAG GTA GGC CAT AAG TTC TGC AAG GAC TTC CCC GGC TTC  1344

CAC CGA GGA GTC CAT CCG GTA TTC AAG ACG TTC CTG AAG GGG CCG AAG  1344

 V   A   P   Q   V   G   H   K   F   C   K   D   F   P   G   F   448


ATC CCA AGC CTC CTC GGA GAC CTC TTG GAG GAG AGA CAG AAG GTA AAG  1392

TAG GGT TCG GAG GAG CCT CTG GAG AAC CTC CTC TCT GTC TTC CAT TTC  1392

 I   P   S   L   L   G   D   L   L   E   E   R   Q   K   V   K   464

AAG AAG ATG AAG GCC ACT ATA GAC CCA ATC GAG AAG AAA CTC CTC GAT  1440

TTC TTC TAC TTC CGG TGA TAT CTG GGT TAG CTC TTC TTT GAG GAG CTA  1440

 K   K   M   K   A   T   I   D   P   I   E   K   K   L   L   D   480


TAC AGG CAA CGA GCA ATC AAA ATC CTT GCT AAT AGC TTC TAC GGT TAC  1488

ATG TCC GTT GCT CGT TAG TTT TAG GAA CGA TTA TCG AAG ATG CCA ATG  1488

 Y   R   Q   R   A   I   K   I   L   A   N   S   F   Y   G   Y   496


TAC GGC TAT ACA AAG GCC CGC TGG TAC TAC AAG GAG TGC GCC GAG AGC  1536

ATG CCG ATA TGT TTC CGG GCG ACC ATG ATG TTC CTC ACG CGG CTC TCG  1536

 Y   G   Y   T   K   A   R   W   Y   Y   K   E   C   A   E   S   512


GTT ACC GGT TGG GGC AGG GAG TAC ATC GAG ACC ACG ATA AGG GAA ATA  1584

CAA TGG CCA ACC CCG TCC CTC ATG TAG CTC TGG TGC TAT TCC CTT TAT  1584

 V   T   G   W   G   R   E   Y   I   E   T   T   I   R   E   I   528


GAG GAG AAA TTT GGC TTT AAA GTC CTC TAC GCG GAC ACA GAT GGA TTT  1632

CTC CTC TTT AAA CCG AAA TTT CAG GAG ATG CGC CTG TGT CTA CCT AAA  1632

 E   E   K   F   G   F   K   V   L   Y   A   D   T   D   G   F   544


TTC GCA ACA ATA CCT GGA GCG GAC GCC GAA ACC GTC AAA AAG AAG GCA  1680

AAG CGT TGT TAT GGA CCT CGC CTG CGG CTT TGG CAG TTT TTC TTC CGT  1680

 F   A   T   I   P   G   A   D   A   E   T   V   K   K   K   A   560
```

```
AAG GAG TTC CTG GAC TAC ATC AAC GCC AAA CTG CCC GGC CTG CTC GAA    1728

TTC CTC AAG GAC CTG ATG TAG TTG CGG TTT GAC GGG CCG GAC GAG CTT    1728

 K   E   F   L   D   Y   I   N   A   K   L   P   G   L   L   E     576


CTC GAA TAC GAG GGC TTC TAC AAG CGC GGC TTC TTC GTG ACG AAG AAG    1776

GAG CTT ATG CTC CCG AAG ATG TTC GCG CCG AAG AAG CAC TGC TTC TTC    1776

 L   E   Y   E   G   F   Y   K   R   G   F   F   V   T   K   K     592


AAG TAC GCG GTT ATA GAC GAG GAG GAC AAG ATA ACG ACG CGC GGG CTT    1824

TTC ATG CGC CAA TAT CTG CTC CTC CTG TTC TAT TGC TGC GCG CCC GAA    1824

 K   Y   A   V   I   D   E   E   D   K   I   T   T   R   G   L     608


GAA ATA GTT AGG CGT GAC TGG AGC GAG ATA GCG AAG GAG ACG CAG GCG    1872

CTT TAT CAA TCC GCA CTG ACC TCG CTC TAT CGC TTC CTC TGC GTC CGC    1872

 E   I   V   R   R   D   W   S   E   I   A   K   E   T   Q   A     624


AGG GTT CTT GAG GCG ATA CTA AAG CAC GGT GAC GTT GAA GAA GCG GTA    1920

TCC CAA GAA CTC CGC TAT GAT TTC GTG CCA CTG CAA CTT CTT CGC CAT    1920

 R   V   L   E   A   I   L   K   H   G   D   V   E   E   A   V     640


AGG ATT GTC AAA GAG GTT ACG GAG AAG CTG AGC AAG TAC GAG GTT CCA    1968

TCC TAA CAG TTT CTC CAA TGC CTC TTC GAC TCG TTC ATG CTC CAA GGT    1968

 R   I   V   K   E   V   T   E   K   L   S   K   Y   E   V   P     656


CCG GAG AAG CTG GTC ATC TAC GAG CAG ATA ACC CGC GAC CTG AAG GAC    2016

GGC CTC TTC GAC CAG TAG ATG CTC GTC TAT TGG GCG CTG GAC TTC CTG    2016

 P   E   K   L   V   I   Y   E   Q   I   T   R   D   L   K   D     672
```

```
TAC AAG GCC ACC GGG CCG CAT GTG GCT GTT GCA AAA CGC CTC GCC GCA    2064

ATG TTC CGG TGG CCC GGC GTA CAC CGA CAA CGT TTT GCG GAG CGG CGT    2064

 Y   K   A   T   G   P   H   V   A   V   A   K   R   L   A   A      688


AGG GGG ATA AAA ATC CGG CCC GGA ACG GTC ATA AGC TAC ATC GTG CTC    2112

TCC CCC TAT TTT TAG GCC GGG CCT TGC CAG TAT TCG ATG TAG CAC GAG    2112

 R   G   I   K   I   R   P   G   T   V   I   S   Y   I   V   L      704


AAA GGC TCG GGA AGG ATT GGG GAC AGG GCT ATA CCC TTT GAC GAA TTT    2160

TTT CCG AGC CCT TCC TAA CCC CTG TCC CGA TAT GGG AAA CTG CTT AAA    2160

 K   G   S   G   R   I   G   D   R   A   I   P   F   D   E   F      720


GAC CCG GCA AAG CAC AAG TAC GAT GCA GAA TAC TAC ATC GAG AAC CAG    2208

CTG GGC CGT TTC GTG TTC ATG CTA CGT CTT ATG ATG TAG CTC TTG GTC    2208

 D   P   A   K   H   K   Y   D   A   E   Y   Y   I   E   N   Q      736


GTT CTT CCA GCT GTG GAG AGG ATT CTG AGG GCC TTT GGT TAC CGT AAA    2256

CAA GAA GGT CGA CAC CTC TCC TAA GAC TCC CGG AAA CCA ATG GCA TTT    2256

 V   L   P   A   V   E   R   I   L   R   A   F   G   Y   R   K      752


GAA GAT TTA AGG TAT CAG AAA ACG CGG CAG GTT GGC TTG GGG GCG TGG    2304

CTT CTA AAT TCC ATA GTC TTT TGC GCC GTC CAA CCG AAC CCC CGC ACC    2304

 E   D   L   R   Y   Q   K   T   R   Q   V   G   L   G   A   W      768


CTA AAA CCT AAG ACA TGA                                            2322

GAT TTT GGA TTC TGT ACT                                            2322

 L   K   P   K   T   *                                             773
```

Figure 4:

Figure 5:

| - dNTPs | | | | | | | + dNTPs | | | | | | |
|---|---|---|---|---|---|---|---|---|---|---|---|---|---|
| 0.4 units | 0.8 units | 1.2 units | 1.6 units | 2 units | 8.5 units | ctrl. 1 | ctrl. 2 | 0.4 units | 0.8 units | 1.2 units | 1.6 units | 2 units | 8.5 units |

| | European Patent Office | EUROPEAN SEARCH REPORT | Application Number<br>EP 96 11 5874 |

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | EP 0 701 000 A (NEW ENGLAND BIOLABS INC.) 13 March 1996<br><br>* page 3, line 10 - line 38 *<br>* page 4, line 54 - page 5, line 44; example 21 *<br>--- | 1-8,10, 14,16, 17,20-24 | C12N15/52 C12N9/12 C12N15/70 C12N1/21 C12P21/00 |
| X | WO 92 09689 A (STRATAGENE) 11 June 1992<br><br><br><br>* page 11, line 26 - page 15, line 19 *<br>* page 19, line 1 - page 25, line 29; examples 3-5,9 *<br>--- | 1,2, 4-10,14, 16,17, 20,21, 23,24 | |
| X | PROC.NATL.ACAD.SCI.USA, vol. 93, no. 11, 28 May 1996, pages 5281-5285, XP000652319 SOUTHWORTH, M.W. ET AL.: "Cloning of thermostable DNA polymerases from hyperthermophilic marine Archaea with emphasis on Thermococcus sp. 9AN-7 and mutations affecting 3'-5' exonuclease activity"<br>* the whole document *<br>--- | 1,3-8, 10,14, 16,17,20 | |
| X | WO 92 03556 A (CETUS CORPORATION) 5 March 1992<br>* page 2, line 28 - page 3, line 16 *<br>* page 23, line 21 - line 37 *<br>--- | 1,5-9 | |

**TECHNICAL FIELDS SEARCHED** (Int.Cl.6)

C12N
C12P

-/--

The present search report has been drawn up for all claims

| Place of search | Date of completion of the search | Examiner |
|---|---|---|
| MUNICH | 30 June 1997 | Donath, C |

EPO FORM 1503 03.82 (P04C01)

# EP 0 834 570 A1

| | European Patent Office | EUROPEAN SEARCH REPORT | Application Number EP 96 11 5874 |

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | ADVANCES IN PROTEIN CHEMISTRY, RICHARDS,F.M. ET AL. (ED.), ACADEMIC PRESS LONDON, vol. 48, 1996, pages 377-435, XP000654858 PERLER, F.B. ET AL.: "Thermostable DNA Polymerases" * the whole document * | 1-22 | |

TECHNICAL FIELDS SEARCHED (Int.Cl.6)

The present search report has been drawn up for all claims

| Place of search | Date of completion of the search | Examiner |
|---|---|---|
| MUNICH | 30 June 1997 | Donath, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

SEQUENCE LISTING

(1) GENERAL INFORMATION:

    (i) APPLICANT:

        (A) NAME: Boehringer Mannheim GmbH

        (B) STREET: Sandhoferstr. 116

        (C) CITY: Mannheim

        (E) COUNTRY: DE

        (F) POSTAL CODE (ZIP): 68305

        (G) TELEPHONE: 06217595482

        (H) TELEFAX: 06217594457

    (ii) TITLE OF INVENTION: Thermostable nucleic acid polymerase from Thermococcus gorgonarius

    (iii) NUMBER OF SEQUENCES: 9

    (iv) COMPUTER READABLE FORM:

        (A) MEDIUM TYPE: Floppy disk

        (B) COMPUTER: IBM PC compatible

        (C) OPERATING SYSTEM: PC-DOS/MS-DOS

        (D) SOFTWARE: PatentIn Release #1.0, Version #1.30 (EPO)

(2) INFORMATION FOR SEQ ID NO: 1:

    (i) SEQUENCE CHARACTERISTICS:

        (A) LENGTH: 26 base pairs

        (B) TYPE: nucleic acid

        (C) STRANDEDNESS: single

        (D) TOPOLOGY: linear

    (ii) MOLECULE TYPE: other nucleic acid

        (A) DESCRIPTION:   /desc = "oligonucleotide"

(xi) SEQUENCE DESCRIPTION: SEQ ID NO: 1:

ATGATHYTNG AYACNGAYTA YATHAC                                              26

(2) INFORMATION FOR SEQ ID NO: 2:

    (i) SEQUENCE CHARACTERISTICS:
       (A) LENGTH: 24 base pairs
       (B) TYPE: nucleic acid
       (C) STRANDEDNESS: single
       (D) TOPOLOGY: linear

    (ii) MOLECULE TYPE: other nucleic acid
       (A) DESCRIPTION:    /desc = "oligonucleotide"

    (xi) SEQUENCE DESCRIPTION: SEQ ID NO: 2:

GGCCTACGAG AGGAACGAAC TGGC                                                24

(2) INFORMATION FOR SEQ ID NO: 3:

    (i) SEQUENCE CHARACTERISTICS:
       (A) LENGTH: 18 base pairs
       (B) TYPE: nucleic acid
       (C) STRANDEDNESS: single
       (D) TOPOLOGY: linear

    (ii) MOLECULE TYPE: other nucleic acid
       (A) DESCRIPTION:    /desc = "oligonucleotide"

    (xi) SEQUENCE DESCRIPTION: SEQ ID NO: 3:

GGCGTAGATG TAGGGCTC                                                       18

(2) INFORMATION FOR SEQ ID NO: 4:

    (i) SEQUENCE CHARACTERISTICS:

        (A) LENGTH: 42 base pairs

        (B) TYPE: nucleic acid

        (C) STRANDEDNESS: single

        (D) TOPOLOGY: linear

    (ii) MOLECULE TYPE: other nucleic acid

        (A) DESCRIPTION:   /desc = "oligonucleotide"

    (xi) SEQUENCE DESCRIPTION: SEQ ID NO: 4:

GAGCTGGTCG AATTCATGAT CCTGGACGCT GACTACATCA CC         42

(2) INFORMATION FOR SEQ ID NO: 5:

    (i) SEQUENCE CHARACTERISTICS:

        (A) LENGTH: 33 base pairs

        (B) TYPE: nucleic acid

        (C) STRANDEDNESS: single

        (D) TOPOLOGY: linear

    (ii) MOLECULE TYPE: other nucleic acid

        (A) DESCRIPTION:   /desc = "oligonucleotide"

    (xi) SEQUENCE DESCRIPTION: SEQ ID NO: 5:

AGCCTGCAGT CATGTCTTAG GTTTTAGCCA CGC         33

(2) INFORMATION FOR SEQ ID NO: 6:

    (i) SEQUENCE CHARACTERISTICS:

        (A) LENGTH: 2322 base pairs

        (B) TYPE: nucleic acid

        (C) STRANDEDNESS: double

        (D) TOPOLOGY: linear

(ii) MOLECULE TYPE: DNA (genomic)

(ix) FEATURE:
    (A) NAME/KEY: CDS
    (B) LOCATION:1..2322

(xi) SEQUENCE DESCRIPTION: SEQ ID NO: 6:

```
ATG ATC CTC GAT ACA GAC TAC ATA ACT GAG GAT GGA AAG CCC GTC        45
Met Ile Leu Asp Thr Asp Tyr Ile Thr Glu Asp Gly Lys Pro Val
 1               5                   10                  15


ATC AGG ATC TTC AAG AAG GAG AAC GGC GAG TTC ACC ATA GAC TAC        90
Ile Arg Ile Phe Lys Lys Glu Asn Gly Glu Phe Thr Ile Asp Tyr
                20                  25                  30


GAC AGA AAC TTT GAG CCA TAC ATC TAC GCG CTC TTG AAG GAC GAC       135
Asp Arg Asn Phe Glu Pro Tyr Ile Tyr Ala Leu Leu Lys Asp Asp
                35                  40                  45


TCT CCG ATT GAG GAC GTC AAG AAG ATA ACT GCC GAG AGG CAC GGC       180
Ser Pro Ile Glu Asp Val Lys Lys Ile Thr Ala Glu Arg His Gly
                50                  55                  60


ACT ACC GTT AGG GTT GTC AGG GCC GAG AAA GTG AAG AAG AAG TTC       225
Thr Thr Val Arg Val Val Arg Ala Glu Lys Val Lys Lys Lys Phe
                65                  70                  75


CTA GGC AGG CCG ATA GAG GTC TGG AAG CTC TAC TTC ACT CAC CCC       270
Leu Gly Arg Pro Ile Glu Val Trp Lys Leu Tyr Phe Thr His Pro
                80                  85                  90


CAG GAC GTT CCC GCA ATC AGG GAC AAG ATA AAG GAG CAT CCT GCC       315
Gln Asp Val Pro Ala Ile Arg Asp Lys Ile Lys Glu His Pro Ala
                95                  100                 105
```

```
GTT GTG GAC ATC TAC GAG TAC GAC ATC CCC TTC GCG AAG CGC TAC          360
Val Val Asp Ile Tyr Glu Tyr Asp Ile Pro Phe Ala Lys Arg Tyr
                110                 115                 120

CTC ATA GAC AAA GGC TTA ATC CCG ATG GAG GGC GAC GAG GAA CTT          405
Leu Ile Asp Lys Gly Leu Ile Pro Met Glu Gly Asp Glu Glu Leu
                125                 130                 135

AAG ATG CTC GCC TTC GAC ATC GAG ACG CTC TAT CAC GAG GGC GAG          450
Lys Met Leu Ala Phe Asp Ile Glu Thr Leu Tyr His Glu Gly Glu
                140                 145                 150

GAG TTC GCC GAA GGG CCT ATC CTG ATG ATA AGC TAC GCC GAC GAG          495
Glu Phe Ala Glu Gly Pro Ile Leu Met Ile Ser Tyr Ala Asp Glu
                155                 160                 165

GAA GGG GCG CGC GTT ATT ACC TGG AAG AAT ATC GAC CTT CCC TAT          540
Glu Gly Ala Arg Val Ile Thr Trp Lys Asn Ile Asp Leu Pro Tyr
                170                 175                 180

GTC GAC GTC GTT TCC ACC GAG AAG GAG ATG ATA AAG CGC TTC CTC          585
Val Asp Val Val Ser Thr Glu Lys Glu Met Ile Lys Arg Phe Leu
                185                 190                 195

AAG GTC GTC AAG GAA AAG GAT CCC GAC GTC CTC ATA ATC TAC AAC          630
Lys Val Val Lys Glu Lys Asp Pro Asp Val Leu Ile Ile Tyr Asn
                200                 205                 210

GGC GAC AAC TTC GAC TTC GCC TAC CTC AAG AAG CGC TCC GAG AAG          675
Gly Asp Asn Phe Asp Phe Ala Tyr Leu Lys Lys Arg Ser Glu Lys
                215                 220                 225

CTC GGA GTC AAG TTC ATC CTC GGA AGG GAA GGG AGC GAA CCG AAA          720
Leu Gly Val Lys Phe Ile Leu Gly Arg Glu Gly Ser Glu Pro Lys
                230                 235                 240
```

```
ATC CAG CGC ATG GGC GAT CGC TTT GCG GTG GAG GTC AAG GGA AGG          765
Ile Gln Arg Met Gly Asp Arg Phe Ala Val Glu Val Lys Gly Arg
                245             250             255

ATT CAC TTC GAC CTC TAC CCC GTC ATT AGG AGA ACG ATT AAC CTC          810
Ile His Phe Asp Leu Tyr Pro Val Ile Arg Arg Thr Ile Asn Leu
                260             265             270

CCC ACT TAC ACC CTT GAG GCA GTA TAT GAA GCC ATC TTT GGA CAG          855
Pro Thr Tyr Thr Leu Glu Ala Val Tyr Glu Ala Ile Phe Gly Gln
                275             280             285

CCG AAG GAG AAG GTC TAC GCT GAG GAG ATA GCG CAG GCC TGG GAA          900
Pro Lys Glu Lys Val Tyr Ala Glu Glu Ile Ala Gln Ala Trp Glu
                290             295               300

ACG GGC GAG GGA TTA GAA AGG GTG GCC CGC TAC TCG ATG GAG GAC          945
Thr Gly Glu Gly Leu Glu Arg Val Ala Arg Tyr Ser Met Glu Asp
                305             310             315

GCG AAG GTA ACC TAT GAA CTC GGA AAA GAG TTC TTC CCT ATG GAA          990
Ala Lys Val Thr Tyr Glu Leu Gly Lys Glu Phe Phe Pro Met Glu
                320             325             330

GCC CAG CTC TCG CGC CTC GTA GGC CAG AGC CTC TGG GAT GTA TCT          1035
Ala Gln Leu Ser Arg Leu Val Gly Gln Ser Leu Trp Asp Val Ser
                335             340             345

CGC TCG AGT ACC GGA AAC CTC GTC GAG TGG TTT TTG CTG AGG AAG          1080
Arg Ser Ser Thr Gly Asn Leu Val Glu Trp Phe Leu Leu Arg Lys
                350             355               360

GCC TAC GAG AGG AAT GAA CTT GCA CCA AAC AAG CCG GAC GAG AGG          1125
Ala Tyr Glu Arg Asn Glu Leu Ala Pro Asn Lys Pro Asp Glu Arg
                365             370             375
```

```
GAG CTG GCA AGA AGA AGG GAG AGC TAC GCG GGT GGA TAC GTC AAG        1170
Glu Leu Ala Arg Arg Arg Glu Ser Tyr Ala Gly Gly Tyr Val Lys
            380             385             390

GAG CCC GAA AGG GGA CTG TGG GAG AAC ATC GTG TAT CTG GAC TTC        1215
Glu Pro Glu Arg Gly Leu Trp Glu Asn Ile Val Tyr Leu Asp Phe
            395             400             405

CGC TCC CTG TAT CCT TCG ATA ATA ATC ACC CAT AAC GTC TCC CCT        1260
Arg Ser Leu Tyr Pro Ser Ile Ile Ile Thr His Asn Val Ser Pro
            410             415             420

GAT ACA CTC AAC AGG GAG GGT TGT GAG GAG TAC GAC GTG GCT CCT        1305
Asp Thr Leu Asn Arg Glu Gly Cys Glu Glu Tyr Asp Val Ala Pro
            425             430             435

CAG GTA GGC CAT AAG TTC TGC AAG GAC TTC CCC GGC TTC ATC CCA        1350
Gln Val Gly His Lys Phe Cys Lys Asp Phe Pro Gly Phe Ile Pro
            440             445             450

AGC CTC CTC GGA GAC CTC TTG GAG GAG AGA CAG AAG GTA AAG AAG        1395
Ser Leu Leu Gly Asp Leu Leu Glu Glu Arg Gln Lys Val Lys Lys
            455             460             465

AAG ATG AAG GCC ACT ATA GAC CCA ATC GAG AAG AAA CTC CTC GAT        1440
Lys Met Lys Ala Thr Ile Asp Pro Ile Glu Lys Lys Leu Leu Asp
            470             475             480

TAC AGG CAA CGA GCA ATC AAA ATC CTT GCT AAT AGC TTC TAC GGT        1485
Tyr Arg Gln Arg Ala Ile Lys Ile Leu Ala Asn Ser Phe Tyr Gly
            485             490             495

TAC TAC GGC TAT ACA AAG GCC CGC TGG TAC TAC AAG GAG TGC GCC        1530
Tyr Tyr Gly Tyr Thr Lys Ala Arg Trp Tyr Tyr Lys Glu Cys Ala
            500             505             510
```

```
GAG AGC GTT ACC GGT TGG GGC AGG GAG TAC ATC GAG ACC ACG ATA     1575
Glu Ser Val Thr Gly Trp Gly Arg Glu Tyr Ile Glu Thr Thr Ile
            515                 520                 525


AGG GAA ATA GAG GAG AAA TTT GGC TTT AAA GTC CTC TAC GCG GAC     1620
Arg Glu Ile Glu Glu Lys Phe Gly Phe Lys Val Leu Tyr Ala Asp
            530                 535                 540


ACA GAT GGA TTT TTC GCA ACA ATA CCT GGA GCG GAC GCC GAA ACC     1665
Thr Asp Gly Phe Phe Ala Thr Ile Pro Gly Ala Asp Ala Glu Thr
            545                 550                 555


GTC AAA AAG AAG GCA AAG GAG TTC CTG GAC TAC ATC AAC GCC AAA     1710
Val Lys Lys Lys Ala Lys Glu Phe Leu Asp Tyr Ile Asn Ala Lys
            560                 565                 570


CTG CCC GGC CTG CTC GAA CTC GAA TAC GAG GGC TTC TAC AAG CGC     1755
Leu Pro Gly Leu Leu Glu Leu Glu Tyr Glu Gly Phe Tyr Lys Arg
            575                 580                 585


GGC TTC TTC GTG ACG AAG AAG AAG TAC GCG GTT ATA GAC GAG GAG     1800
Gly Phe Phe Val Thr Lys Lys Lys Tyr Ala Val Ile Asp Glu Glu
            590                 595                 600


GAC AAG ATA ACG ACG CGC GGG CTT GAA ATA GTT AGG CGT GAC TGG     1845
Asp Lys Ile Thr Thr Arg Gly Leu Glu Ile Val Arg Arg Asp Trp
            605                 610                 615


AGC GAG ATA GCG AAG GAG ACG CAG GCG AGG GTT CTT GAG GCG ATA     1890
Ser Glu Ile Ala Lys Glu Thr Gln Ala Arg Val Leu Glu Ala Ile
            620                 625                 630


CTA AAG CAC GGT GAC GTT GAA GAA GCG GTA AGG ATT GTC AAA GAG     1935
Leu Lys His Gly Asp Val Glu Glu Ala Val Arg Ile Val Lys Glu
            635                 640                 645
```

```
GTT ACG GAG AAG CTG AGC AAG TAC GAG GTT CCA CCG GAG AAG CTG        1980
Val Thr Glu Lys Leu Ser Lys Tyr Glu Val Pro Pro Glu Lys Leu
            650                 655                 660

GTC ATC TAC GAG CAG ATA ACC CGC GAC CTG AAG GAC TAC AAG GCC        2025
Val Ile Tyr Glu Gln Ile Thr Arg Asp Leu Lys Asp Tyr Lys Ala
            665                 670                 675

ACC GGG CCG CAT GTG GCT GTT GCA AAA CGC CTC GCC GCA AGG GGG        2070
Thr Gly Pro His Val Ala Val Ala Lys Arg Leu Ala Ala Arg Gly
            680                 685                 690

ATA AAA ATC CGG CCC GGA ACG GTC ATA AGC TAC ATC GTG CTC AAA        2115
Ile Lys Ile Arg Pro Gly Thr Val Ile Ser Tyr Ile Val Leu Lys
            695                 700                 705

GGC TCG GGA AGG ATT GGG GAC AGG GCT ATA CCC TTT GAC GAA TTT        2160
Gly Ser Gly Arg Ile Gly Asp Arg Ala Ile Pro Phe Asp Glu Phe
            710                 715                 720

GAC CCG GCA AAG CAC AAG TAC GAT GCA GAA TAC TAC ATC GAG AAC        2205
Asp Pro Ala Lys His Lys Tyr Asp Ala Glu Tyr Tyr Ile Glu Asn
            725                 730                 735

CAG GTT CTT CCA GCT GTG GAG AGG ATT CTG AGG GCC TTT GGT TAC        2250
Gln Val Leu Pro Ala Val Glu Arg Ile Leu Arg Ala Phe Gly Tyr
            740                 745                 750

CGT AAA GAA GAT TTA AGG TAT CAG AAA ACG CGG CAG GTT GGC TTG        2295
Arg Lys Glu Asp Leu Arg Tyr Gln Lys Thr Arg Gln Val Gly Leu
            755                 760                 765

GGG GCG TGG CTA AAA CCT AAG ACA TGA                                2322
Gly Ala Trp Leu Lys Pro Lys Thr  *
            770
```

(2) INFORMATION FOR SEQ ID NO: 7:

    (i) SEQUENCE CHARACTERISTICS:
      (A) LENGTH: 773 amino acids
      (B) TYPE: amino acid
      (D) TOPOLOGY: linear

    (ii) MOLECULE TYPE: protein
    (xi) SEQUENCE DESCRIPTION: SEQ ID NO: 7:

```
Met Ile Leu Asp Thr Asp Tyr Ile Thr Glu Asp Gly Lys Pro Val
 1               5                  10                  15

Ile Arg Ile Phe Lys Lys Glu Asn Gly Glu Phe Thr Ile Asp Tyr
                20                  25                  30

Asp Arg Asn Phe Glu Pro Tyr Ile Tyr Ala Leu Leu Lys Asp Asp
                35                  40                  45

Ser Pro Ile Glu Asp Val Lys Lys Ile Thr Ala Glu Arg His Gly
                50                  55                  60

Thr Thr Val Arg Val Val Arg Ala Glu Lys Val Lys Lys Lys Phe
                65                  70                  75

Leu Gly Arg Pro Ile Glu Val Trp Lys Leu Tyr Phe Thr His Pro
                80                  85                  90

Gln Asp Val Pro Ala Ile Arg Asp Lys Ile Lys Glu His Pro Ala
                95                  100                 105

Val Val Asp Ile Tyr Glu Tyr Asp Ile Pro Phe Ala Lys Arg Tyr
                110                 115                 120

Leu Ile Asp Lys Gly Leu Ile Pro Met Glu Gly Asp Glu Glu Leu
                125                 130                 135
```

```
Lys Met Leu Ala Phe Asp Ile Glu Thr Leu Tyr His Glu Gly Glu
                140                 145                 150

Glu Phe Ala Glu Gly Pro Ile Leu Met Ile Ser Tyr Ala Asp Glu
                155                 160                 165

Glu Gly Ala Arg Val Ile Thr Trp Lys Asn Ile Asp Leu Pro Tyr
                170                 175                 180

Val Asp Val Val Ser Thr Glu Lys Glu Met Ile Lys Arg Phe Leu
                185                 190                 195

Lys Val Val Lys Glu Lys Asp Pro Asp Val Leu Ile Ile Tyr Asn
                200                 205                 210

Gly Asp Asn Phe Asp Phe Ala Tyr Leu Lys Lys Arg Ser Glu Lys
                215                 220                 225

Leu Gly Val Lys Phe Ile Leu Gly Arg Glu Gly Ser Glu Pro Lys
                230                 235                 240

Ile Gln Arg Met Gly Asp Arg Phe Ala Val Glu Val Lys Gly Arg
                245                 250                 255

Ile His Phe Asp Leu Tyr Pro Val Ile Arg Arg Thr Ile Asn Leu
                260                 265                 270

Pro Thr Tyr Thr Leu Glu Ala Val Tyr Glu Ala Ile Phe Gly Gln
                275                 280                 285

Pro Lys Glu Lys Val Tyr Ala Glu Glu Ile Ala Gln Ala Trp Glu
                290                 295                 300

Thr Gly Glu Gly Leu Glu Arg Val Ala Arg Tyr Ser Met Glu Asp
                305                 310                 315
```

```
Ala Lys Val Thr Tyr Glu Leu Gly Lys Glu Phe Phe Pro Met Glu
                320             325             330

Ala Gln Leu Ser Arg Leu Val Gly Gln Ser Leu Trp Asp Val Ser
                335             340             345

Arg Ser Ser Thr Gly Asn Leu Val Glu Trp Phe Leu Leu Arg Lys
                350             355             360

Ala Tyr Glu Arg Asn Glu Leu Ala Pro Asn Lys Pro Asp Glu Arg
                365             370             375

Glu Leu Ala Arg Arg Arg Glu Ser Tyr Ala Gly Gly Tyr Val Lys
                380             385             390

Glu Pro Glu Arg Gly Leu Trp Glu Asn Ile Val Tyr Leu Asp Phe
                395             400             405

Arg Ser Leu Tyr Pro Ser Ile Ile Ile Thr His Asn Val Ser Pro
                410             415             420

Asp Thr Leu Asn Arg Glu Gly Cys Glu Glu Tyr Asp Val Ala Pro
                425             430             435

Gln Val Gly His Lys Phe Cys Lys Asp Phe Pro Gly Phe Ile Pro
                440             445             450

Ser Leu Leu Gly Asp Leu Leu Glu Glu Arg Gln Lys Val Lys Lys
                455             460             465

Lys Met Lys Ala Thr Ile Asp Pro Ile Glu Lys Lys Leu Leu Asp
                470             475             480

Tyr Arg Gln Arg Ala Ile Lys Ile Leu Ala Asn Ser Phe Tyr Gly
                485             490             495
```

Tyr Tyr Gly Tyr Thr Lys Ala Arg Trp Tyr Tyr Lys Glu Cys Ala
500 505 510

Glu Ser Val Thr Gly Trp Gly Arg Glu Tyr Ile Glu Thr Thr Ile
515 520 525

Arg Glu Ile Glu Glu Lys Phe Gly Phe Lys Val Leu Tyr Ala Asp
530 535 540

Thr Asp Gly Phe Phe Ala Thr Ile Pro Gly Ala Asp Ala Glu Thr
545 550 555

Val Lys Lys Lys Ala Lys Glu Phe Leu Asp Tyr Ile Asn Ala Lys
560 565 570

Leu Pro Gly Leu Leu Glu Leu Glu Tyr Glu Gly Phe Tyr Lys Arg
575 580 585

Gly Phe Phe Val Thr Lys Lys Lys Tyr Ala Val Ile Asp Glu Glu
590 595 600

Asp Lys Ile Thr Thr Arg Gly Leu Glu Ile Val Arg Arg Asp Trp
605 610 615

Ser Glu Ile Ala Lys Glu Thr Gln Ala Arg Val Leu Glu Ala Ile
620 625 630

Leu Lys His Gly Asp Val Glu Glu Ala Val Arg Ile Val Lys Glu
635 640 645

Val Thr Glu Lys Leu Ser Lys Tyr Glu Val Pro Pro Glu Lys Leu
650 655 660

Val Ile Tyr Glu Gln Ile Thr Arg Asp Leu Lys Asp Tyr Lys Ala
665 670 675

```
Thr Gly Pro His Val Ala Val Ala Lys Arg Leu Ala Ala Arg Gly
            680                 685                 690


Ile Lys Ile Arg Pro Gly Thr Val Ile Ser Tyr Ile Val Leu Lys
            695                 700                 705


Gly Ser Gly Arg Ile Gly Asp Arg Ala Ile Pro Phe Asp Glu Phe
            710                 715                 720


Asp Pro Ala Lys His Lys Tyr Asp Ala Glu Tyr Tyr Ile Glu Asn
            725                 730                 735


Gln Val Leu Pro Ala Val Glu Arg Ile Leu Arg Ala Phe Gly Tyr
            740                 745                 750


Arg Lys Glu Asp Leu Arg Tyr Gln Lys Thr Arg Gln Val Gly Leu
            755                 760                 765


Gly Ala Trp Leu Lys Pro Lys Thr   *
            770
```

(2) INFORMATION FOR SEQ ID NO: 8:

        (i) SEQUENCE CHARACTERISTICS:
            (A) LENGTH: 34 base pairs
            (B) TYPE: nucleic acid
            (C) STRANDEDNESS: single
            (D) TOPOLOGY: linear


        (ii) MOLECULE TYPE: other nucleic acid
            (A) DESCRIPTION:   /desc = "oligonucleotide"


        (xi) SEQUENCE DESCRIPTION: SEQ ID NO: 8:


AGCTTATCGA TGGCACTTTT CGGGGAAATG TGCG                                    34

(2) INFORMATION FOR SEQ ID NO: 9:

```
        (i) SEQUENCE CHARACTERISTICS:
            (A) LENGTH: 36 base pairs
            (B) TYPE: nucleic acid
            (C) STRANDEDNESS: single
            (D) TOPOLOGY: linear


       (ii) MOLECULE TYPE: other nucleic acid
            (A) DESCRIPTION:   /desc = "oligonucleotide"


       (xi) SEQUENCE DESCRIPTION: SEQ ID NO: 9:


AGCTTATCGA TAAGCGGATG CCGGGAGCAG ACAAGC                                       36
```